(19) Europäisches Patentamt
European Patent Office
Office européen des brevets

(11) EP 1 852 425 A1

(12) **EUROPEAN PATENT APPLICATION**
published in accordance with Art. 158(3) EPC

(43) Date of publication:
**07.11.2007 Bulletin 2007/45**

(21) Application number: **06714447.7**

(22) Date of filing: **23.02.2006**

(51) Int Cl.:
*C07D 263/16* (2006.01) *C07D 263/22* (2006.01)
*C07D 263/24* (2006.01) *C07D 263/52* (2006.01)
*C07D 263/58* (2006.01) *C07D 277/14* (2006.01)
*A01N 43/76* (2006.01) *A01N 43/78* (2006.01)
*A01N 47/02* (2006.01)

(86) International application number:
**PCT/JP2006/303307**

(87) International publication number:
**WO 2006/090792 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.02.2005 JP 2005049564**

(71) Applicant: **NIHON NOHYAKU CO., LTD.**
**Tokyo 103-8236 (JP)**

(72) Inventors:
- **HINO, Tomokazu,**
**c/o Research and Development**
**Kawachinagano-shi,**
**Osaka, 5860094 (JP)**
- **SHIGENARI, Toshihiko,**
**c/o Research and Development**
**Kawachinagano-shi,**
**Osaka, 5860094 (JP)**
- **KIYOKAWA, Takahiro,**
**c/o Research and Development**
**Kawachinagano,**
**Osaka, 5860094 (JP)**
- **MAMETZUKA, Koki,**
**c/o Research and Development**
**Kawachinagano-shi,**
**Osaka, 5860094 (JP)**
- **YAMADA, Yasuko,**
**c/o Research and Development**
**Kawachinagano--shi,**
**Osaka, 5860094 (JP)**

(74) Representative: **Grünecker, Kinkeldey,**
**Stockmair & Schwanhäusser**
**Anwaltssozietät**
**Maximilianstrasse 58**
**80538 München (DE)**

(54) **NOVEL HALOALKYLSULFONANILIDE DERIVATIVE, HERBICIDE, AND METHOD OF USE THEREOF**

(57) A haloalkylsulfonanilide derivative represented by the formula (I): {wherein $R^1$ represents haloalkyl; $R^2$ represents H, alkylcarbonyl, optionally substituted phenylcarbonyl, alkoxycarbonyl, optionally substituted phenoxycarbonyl, optionally substituted phenylsulfonyl, etc.; $R^3$ and $R^4$ each represents H, alkyl, cycloalkyl, halogeno, CN, etc.; $R^5$, $R^6$, $R^7$, and $R^8$ each represents H, halogeno, alkyl, cycloalkyl, optionally substituted phenyl ($C_{1-6}$ alkyl), an optionally substituted heterocycle, etc.; A and W each represents O or S; and X represents one to four substituents selected among H, halogeno, alkyl, alkenyl, optionally substituted phenyl, optionally substituted phenylalkylaminocarbonyl, OH, CN, etc.} and a salt thereof; and a herbicide containing any of these compounds as an active ingredient. The herbicide is excellent in wide applicability, persistency, and selectivity for crops and effectiveness against weeds. It is excellent especially as a herbicide for rice fields.

EP 1 852 425 A1

## Description

### TECHNICAL FIELD

The present invention relates to a novel haloalkyl sulfonanilide derivative or a salt thereof, and to a herbicide containing the compound as an active ingredient and a method of using the same.

### BACKGROUND ART

Compounds in which haloalkyl sulfonanilide and heterocyclic ring are bound through a spacer such as an alkylene group to a nitrogen atom in the heterocyclic ring have been conventionally known as herbicide (for example, see JP-A-2004-107322).

However, as to the haloalkyl sulfonanilide derivative of the present invention, the particular method of production, physicochemical properties of the compound and herbicide activity are not yet known.

### DISCLOSURE OF THE INVENTION

As described above, it has been known that a certain haloalkyl sulfonamide derivative is useful as a herbicide. However, its properties such as efficacy, applicability on wide range of weed species including hard-to-kill weeds, residual activity, and selectivity between crops and weeds are insufficient and development of a herbicide with superior properties has been desired.

In developing a novel herbicide, the present inventors have been working hard on synthesis of derivatives with sulfonanilide structure and studying physiological activity thereof, and have found that a derivative of haloalkyl sulfonanilide represented by the general formula (I) of the present invention is a novel compound hitherto unreported in the literature, possesses properties of both remarkable herbicidal effect and superior selectivity between crops and weeds, compared to the compounds disclosed in the prior art, and useful as a herbicide, in particular herbicide for rice paddy, to complete the present invention.

That is, the present invention relates to a haloalkyl sulfonanilide derivative represented by the general formula (I):

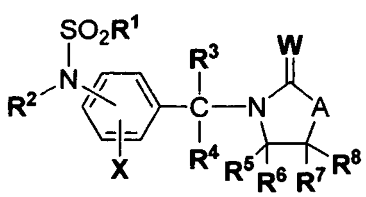

( I )

wherein $R^1$ is a halo ($C_1$-$C_6$) alkyl group;

$R^2$ is hydrogen atom, a ($C_1$-$C_6$) alkoxycarbonyl ($C_1$-$C_6$) alkyl group, a ($C_1$-$C_{18}$) alkylcarbonyl group, a halo($C_1$-$C_6$) alkylcarbonyl group, a phenylcarbonyl group, a substituted phenylcarbonyl group with 1 to 5 substituents which may be the same or different and are selected from Y (Y is described below), a ($C_1$-$C_{18}$) alkoxycarbonyl group, a ($C_2$-$C_{18}$) alkenyloxycarbonyl group, a ($C_2$-$C_{18}$) alkynyloxycarbonyl group, a halo ($C_1$-$C_6$) alkoxycarbonyl group, a ($C_1$-$C_6$) alkoxy ($C_1$-$C_6$) alkoxycarbonyl group, a ($C_1$-$C_6$) alkylthio ($C_1$-$C_6$) alkoxycarbonyl group, a ($C_1$-$C_6$) alkylsulfinyl ($C_1$-$C_6$) alkoxycarbonyl group, a ($C_1$-$C_6$) alkylsulfonyl ($C_1$-$C_6$) alkoxycarbonyl group, a phenoxycarbonyl group, a substituted phenoxycarbonyl group with 1 to 5 substituents which may be the same or different and are selected from Y (Y is described below), a phenoxy ($C_1$-$C_6$) alkylcarbonyl group, a substituted phenoxy ($C_1$-$C_6$) alkylcarbonyl group with 1 to 5 substituents which may be the same or different and are selected from Y (Y is described below), a benzyloxycarbonyl group, a substituted benzyloxycarbonyl group with 1 to 5 substituents which may be the same or different and are selected from Y (Y is described below), a ($C_1$-$C_6$) alkylthiocarbonyl group, a ($C_1$-$C_6$) alkylsulfonyl group, a halo ($C_1$-$C_6$) alkylsulfonyl group, a phenylsulfonyl group or a substituted phenylsulfonyl group with 1 to 5 substituents which may be the same or different and are selected from Y (Y is described below);

$R^3$ and $R^4$ may be the same or different, and each are a hydrogen atom, a ($C_1$-$C_6$) alkyl group, a ($C_3$-$C_6$) cycloalkyl group, a ($C_1$-$C_6$) alkoxy group, a halogen atom or a cyano group; and $R^3$ and $R^4$ may also be mutually bonded to form a 3- to 7-membered ring;

$R^5$, $R^6$, $R^7$ and $R^8$ may be the same or different and are a hydrogen atom, a halogen atom, a ($C_1$-$C_6$) alkyl

group, a ($C_3$-$C_6$) cycloalkyl group, a ($C_1$-$C_6$) alkoxy group, a halo ($C_1$-$C_6$) alkyl group, a ($C_1$-$C_6$) alkoxy ($C_1$-$C_6$) alkyl group, a ($C_1$-$C_6$) alkylcarbonyloxy ($C_1$-$C_6$) alkyl group, a mono ($C_1$-$C_6$) alkylamino ($C_1$-$C_6$) alkyl group, a di ($C_1$-$C_6$) alkylamino ($C_1$-$C_6$) alkyl group in which the alkyl groups may be the same or different, a mono ($C_1$-$C_6$) alkylaminocarbonyl ($C_1$-$C_6$) alkyl group, a di ($C_1$-$C_6$) alkylaminocarbonyl ($C_1$-$C_6$) alkyl group in which the alkyl groups may be the same or different, a phenyl ($C_1$-$C_6$) alkyl group, a substituted phenyl ($C_1$-$C_6$) alkyl group with 1 to 5 substituents on the ring which may be the same or different and are selected from Y (Y is described below), a phenoxy ($C_1$-$C_6$) alkyl group, a substituted phenoxy ($C_1$-$C_6$) alkyl group with 1 to 5 substituents on the ring which may be the same or different and are selected from Y (Y is described below), a phenyl group, a substituted phenyl group with 1 to 5 substituents which may be the same or different and are selected from Y (Y is described below), a heterocyclic group (wherein the heterocyclic group is a pyridyl group, a pyridine-N-oxide group, a pyrimidinyl group, a pyradinyl group, a triadinyl group, a furyl group, a tetrahydrofuryl group, a thienyl group, a tetrahydrothienyl group, a tetrahydropyranyl group, a tetrahydrothiopyranyl group, an oxazolyl group, an isoxazolyl group, an oxadiazolyl group, a thiazolyl group, an isothiazolyl group, a thiadiazolyl group, an imidazolyl group, a triazolyl group, a pyrazolyl group or a pyrolydinyl group), a substituted heterocyclic group (wherein the heterocyclic group is the same as described above) with one or more substituents which may be the same or different and are selected from Y (Y is described below), a ($C_1$-$C_6$) alkoxycarbonyl group, a mono ($C_1$-$C_6$) alkylaminocarbonyl group, a di ($C_1$-$C_6$) alkylaminocarbonyl group in which the alkyl groups may be the same or different, a hydroxyl group or a cyano group; and $R^5$ and $R^6$, $R^5$ and $R^7$, $R^5$ and $R^8$, $R^6$ and $R^7$, $R^6$ and $R^8$, and, $R^7$ and $R^8$, may be mutually bonded to form a 3- to 7-membered ring, $R^5$ and $R^6$, and $R^7$ and $R^8$ may form a carbonyl group, and $R^6$ and $R^7$ may form a double bond;

A is an oxygen atom or a sulfur atom;
W is an oxygen atom or a sulfur atom;

X may be the same or different and is 1 to 4 substituents selected from the group consisting of a hydrogen atom, a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkoxy ($C_1$-$C_6$) alkyl group, a halo ($C_1$-$C_6$) alkoxy ($C_1$-$C_6$) alkyl group, a ($C_1$-$C_6$) alkylthio group, a halo ($C_1$-$C_6$) alkylthio group, a ($C_1$-$C_6$) alkylthio ($C_1$-$C_6$) alkyl group, a halo ($C_1$-$C_6$) alkylthio ($C_1$-$C_6$) alkyl group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a phenyl group, a substituted phenyl group with 1 to 5 substituents which may be the same or different and are selected from Y (Y is described below), a phenoxy group, a substituted phenoxy group with 1 to 5 substituents which may be the same or different and are selected from Y (Y is described below), a phenylthio group, a substituted phenylthio group with 1 to 5 substituents which may be the same or different and are selected from Y (Y is described below), a phenylsulfinyl group, a substituted phenylsulfinyl group with 1 to 5 substituents which may be the same or different and are selected from Y (Y is described below), a phenylsulfonyl group, a substituted phenylsulfonyl group with 1 to 5 substituents which may be the same or different and are selected from Y (Y is described below), a ($C_1$-$C_6$) alkylcarbonyl group, a halo ($C_1$-$C_6$) alkylcarbonyl group, a phenylcarbonyl group, a substituted phenylcarbonyl group with 1 to 5 substituents which may be the same or different and are selected from Y (Y is described below), a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a phenylaminocarbonyl group, a substituted phenylaminocarbonyl group with 1 to 5 substituents on the ring which may be the same or different and are selected from Y (Y is described below), a phenyl ($C_1$-$C_6$) alkylaminocarbonyl group, a substituted phenyl ($C_1$-$C_6$) alkylaminocarbonyl group with 1 to 5 substituents on the ring which may be the same or different and are selected from Y (Y is described below), a hydroxyl group and a cyano group; X, together with an adjacent carbon atom on the benzene ring, may also form a 5- or 6-membered ring with a ($C_1$-$C_4$) alkylene group which may be interrupted by 1 or 2 hetero atoms which may be the same or different and are selected from the group consisting of an oxygen atom, a sulfur atom and a nitrogen atom (wherein the nitrogen atom may be substituted with a hydrogen atom, a ($C_1$-$C_6$) alkyl group, a ($C_2$-$C_6$) alkenyl group, a ($C_2$-$C_6$) alkynyl group or a cyclo ($C_3$-$C_6$) alkyl group);

Y may be the same or different and is 1 to 5 substituents selected from the group consisting of a halogen atom; a ($C_1$-$C_6$) alkyl group; a ($C_2$-$C_6$) alkenyl group; a ($C_2$-$C_6$) alkynyl group; a cyclo ($C_3$-$C_6$) alkyl group; a halo ($C_1$-$C_6$) alkyl group; a cyclohalo ($C_3$-$C_6$) alkyl group; a ($C_1$-$C_6$) alkoxy group; a halo ($C_1$-$C_6$) alkoxy group; a ($C_1$-$C_6$) alkylthio group; a halo ($C_1$-$C_6$) alkylthio group; a ($C_1$-$C_6$) alkylsulfinyl group; a halo ($C_1$-$C_6$) alkylsulfinyl group; a ($C_1$-$C_6$) alkylsulfonyl group; a halo ($C_1$-$C_6$) alkylsulfonyl group; a phenyl group; a substituted phenyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may

be the same or different, a hydroxyl group and a cyano group; a heterocyclic group (wherein the heterocyclic group is the same as described above); a substituted heterocyclic group (wherein the heterocyclic group is the same as described above) with one or more substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group;

a phenoxy group; a substituted phenoxy group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenylthio group; a substituted phenylthio group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenylsulfinyl group; a substituted phenylsulfinyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group;

a phenylsulfonyl group; a substituted phenylsulfonyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a ($C_1$-$C_6$) alkylcarbonyl group; a halo ($C_1$-$C_6$) alkylcarbonyl group; a phenylcarbonyl group; a substituted phenylcarbonyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group;

a ($C_1$-$C_6$) alkoxycarbonyl group; a carboxyl group; a mono ($C_1$-$C_6$) alkylaminocarbonyl group; a di ($C_1$-$C_6$) alkylaminocarbonyl group in which the alkyl groups may be the same or different; a phenylaminocarbonyl group; a substituted phenylaminocarbonyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenyl ($C_1$-$C_6$) alkylaminocarbonyl group; a substituted phenyl ($C_1$-$C_6$) alkylaminocarbonyl group with 1 to 5 substituents which may be the same or different and are selected from the group consisting

of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a hydroxyl group; and a cyano group; and Y, together with an adjacent carbon atom or nitrogen atom on the benzene ring or heterocyclic ring, may form 5-or 6-membered ring with a ($C_1$-$C_4$) alkylene group which may be interrupted by 1 or 2 hetero atoms which may be the same or different and are selected from the group consisting of an oxygen atom, a sulfur atom and a nitrogen atom (wherein the nitrogen atom may be substituted with a hydrogen atom, a ($C_1$-$C_6$) alkyl group, a ($C_2$-$C_6$) alkenyl group, a ($C_2$-$C_6$) alkynyl group or a cyclo ($C_3$-$C_6$) alkyl group), or a salt thereof, a herbicide containing the compound as an active ingredient, and a method of using the same.

The present invention provides a novel compound having superior properties such as applicability on wide range of weed species including hard-to-kill weeds, residual activity, and selectivity between crops and weeds, and which is particularly useful as a herbicide for rice paddy.

BEST MODE FOR CARRYING OUT THE INVENTION

In the definition of the general formula (I) of the haloalkyl sulfonanilide derivatives of the present invention, "halogen atom" represents chlorine atom, bromine atom, iodine atom or fluorine atom. "($C_1$-$C_6$) alkylene group" represents a linear or branched chain alkylene group with 1 to 6 carbon atoms such as a methylene group, ethylene group, propylene group, dimethylmethylene group, tetramethylene group, isobutylene group, dimethylethylene group and hexamethylene group; "($C_2$-$C_6$) alkenylene group" represents a linear or branched chain alkenylene group with 2 to 6 carbon atoms. "($C_1$-$C_6$) alkyl group" represents a linear or branched chain alkyl group with 1 to 6 carbon atoms, for example, a methyl group, ethyl group, n-propyl group, isopropyl group, n-butyl group, isobutyl group, sec-butyl group, t-butyl group, n-pentyl group, neopentyl group, or n-hexyl group. "Halo ($C_1$-$C_6$) alkyl group" represents a linear or branched chain alkyl group with 1 to 6 carbon atoms which is substituted with one or more halogen atoms that may be the same or different, for example, a trifluoromethyl group, difluoromethyl group, perfluoroethyl group, perfloroisopropyl group, chloromethyl group, bromomethyl group, 1-bromoethyl group, or 2,3-dibromopropyl group. "($C_3$-$C_6$) cycloalkyl group" represents an alicyclic alkyl group with 3 to 6 carbon atoms such as a cyclopropyl group, cyclobutyl group, cyclopentyl group, cyclohexyl group, 2-methylcyclopropyl group, or 2-methylcyclopentyl group.

"($C_1$-$C_6$) alkoxy group" represents a linear or branched chain alkoxy group with 1 to 6 carbon atoms, for example, a methoxy group, ethoxy group, normal propoxy group, isopropoxy group, n-butoxy group, secondary butoxy group, tertiary butoxy group, n-pentyloxy group, isopentyloxy group, neopentyloxy group, or n-hexyloxy group. "Halo ($C_1$-$C_6$) alkoxy group" represents a linear or branched chain alkoxy group with 1 to 6 carbon atoms substituted with one or more halogen atoms that may be the same or different, for example, a difluoromethoxy group, trifluoromethoxy group, or 2,2,2-trifluoroethoxy group. "($C_1$-$C_6$) alkoxycarbonyl group" represents a linear or branched chain alkoxycarbonyl group with 1 to 6 carbon atoms, for example, a methoxycarbonyl group, ethoxycarbonyl group, n-propoxycarbonyl group, isopropoxycarbonyl group, n-butoxycarbonyl group, or t-butoxycarbonyl group. "($C_1$-$C_6$) alkylthio group" represents a linear or branched chain alkylthio group with 1 to 6 carbon atoms, for example, a methylthio group, ethylthio group, n-propylthio group, isopropylthio group n-butylthio group, sec-butylthio group, t-butylthio group, n-pentylthio group, isopentylthio group, or n-hexylthio group. "($C_1$-$C_6$) alkylsulfinyl group" represents a linear or branched chain alkylsulfinyl group with 1 to 6 carbon atoms, for example, a methylsulfinyl group, ethylsulfinyl group, n-propylsulfinyl group, isopropylsulfinyl group, n-butylsulfinyl group, sec-butylsulfinyl group, t-butylsulfinyl group, n-pentylsulfinyl group, isopentylsulfinyl group, or n-hexylsulfinyl group. "($C_1$-$C_6$) alkylsulfonyl group" represents a linear or branched chain alkylsulfonyl group with 1 to 6 carbon atoms, for example, a methylsulfonyl group, ethylsulfonyl group, n-propylsulfonyl group, isopropylsulfonyl group, n-butylsulfonyl group, sec-butylsulfonyl group, t-butylsulfonyl group, n-pentylsulfonyl group, isopentylsulfonyl group, or n-hexylsulfonyl group.

Also, "($C_1$-$C_6$)", " ($C_3$-$C_6$) ", " ($C_2$-$C_{18}$) " and the like represent the range of the numbers of carbon atoms in various substituents.

Examples of the salts of haloalkyl sulfonamide derivative of the present invention represented by the general formula (I) include salts with alkali metal salts of sodium ion, potassium ion and the like, and alkali earth metal salts of calcium ion and the like. Further, the salts may be hydrates.

Haloalkyl sulfonanilide derivatives of the present invention represented by the general formula (I) may contain one or a plurality of asymmetric centers in the structural formula and in some cases may contain two or more optical isomers and diastereomer, but the present invention includes each optical isomer and even a mixture containing the optical isomers at an optional ratio.

Following is schematic drawings of the representative Production Process of production in the present invention but the

present invention is not limited to these.

Production Process 1

Haloalkyl sulfonamide derivative of the present invention represented by the general formula (I) may be produced by the following Production Process.

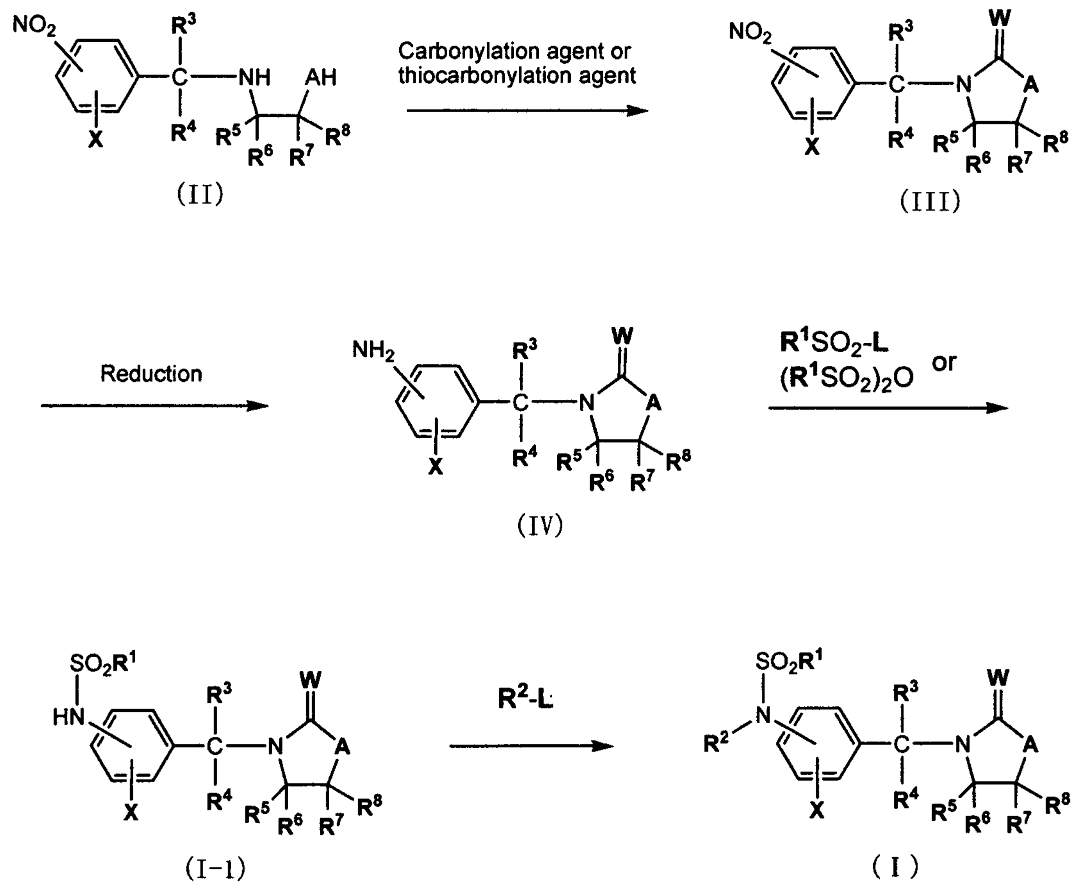


(II) (III) (IV) (I-1) (I)

wherein $R^1$, $R^2$, $R^3$, $R^4$, $R^5$, $R^6$, $R^7$, $R^8$, A, X and W are the same as described before, and L represents a leaving group such as a halogen atom.

By reacting a nitro compound represented by the general formula (II) with a carbonylation agent such as phosgene or a thiocarbonylation agent such as thiophosgene in the presence or absence of a base and an inert solvent, a cyclic nitro compound represented by the general formula (III) is obtained. By reducing the nitro group with or without isolating this cyclic nitro compound, an aniline compound represented by the general formula (IV) is obtained. By reacting this aniline compound with or without isolation with a haloalkylsulfonyl derivative represented by $R^1SO_2$-L or $(R^1SO_2)_2O$, haloalkyl sulfonamide derivatives may be produced which are a part of the compound of the present invention and is represented by the general formula (I-1) (when $R^2$ is hydrogen atom in the general formula (I)). Further, by reacting the haloalkyl sulfonamide derivatives represented by the general formula (I-1) with or without isolation with a compound represented by the general formula $R^2$-L, the haloalkyl sulfonamide derivative of the present invention which is represented by the general formula (I) may be produced.

1-1) General formula (II) → General formula (III)

In this reaction any inert solvent that does not strongly inhibit the proceeding of the reaction may be used. Inert solvent may include, for example: linear or cyclic chain ethers such as diethyl ether, tetrahydrofuran, and dioxane; aromatic hydrocarbons such as benzene, toluene, and xylene; halogenated hydrocarbons such as methylene chloride, chloroform, and tetrachloromethane; and halogenated aromatic hydrocarbons such as chlorobenzene and dichlorobenzene. These inert solvents may be used singly or in a mixture of two or more. A carbonylation agent that may be used

in the present reaction includes, for example, phosgene, diphosgene, triphosgene, diethylcarbonate, and 1-1'-carbonyldiimidazole. Examples of thiocarbonylation agent may include thiophosgene and 1,1'-thiocarbonyldiimidazole. The amount of carbonylation agent or thiocarbonylation agent to be used may be chosen appropriately in the range of 0.3 to 10 times mol to the nitro-compound represented by the general formula (II). Examples of bases that may be used in the present reaction include: nitrogen-containing organic bases such as triethylamine, diisopropylethylamine, 1,8-diazabicyclo[5,4,0]-undec-7-ene, and pyridine; inorganic bases such as sodium carbonate, potassium carbonate, sodium bicarbonate, sodium hydroxide, potassium hydroxide, sodium hydride and sodium metal; organic bases such as sodium acetate, and potassium acetate; alcoholates such as sodium ethoxide and potassium t-butoxide. The amount of the base to be used may be chosen appropriately in the range of 0.5 to 5 times mol to the nitro-compound represented by the general formula (II). The reaction temperature may be chosen in the range of 0 to 150°C, and the reaction time may not be constant depending on the reaction scale, temperature and the like but may be chosen in the range of a few min to 48 hours. After the completion of the reaction, the target compound may be isolated from the reaction mixture that contains the target compound by a standard method, and the target compound may be produced by purifying, as necessary, by recrystallization, distillation, column chromatography or the like. Also, after completion of the reaction the target compound may be used in the next reaction without isolating.

1-2) General formula (III) → General formula (IV)

Examples of inert solvents that may be used in this reaction include: alcohols such as methanol and ethanol; ethers such as tetrahydrofuran and dioxane; and water, and these may be used singly or as a mixture of two or more. Also, it may be possible to use an aqueous solution of acid that is used as a reducing agent shown next as an inert solvent. Examples of reducing agents that may be used in the present reaction include metal-acid system and metal-salts system. Examples of the metals include iron, tin, and zinc. Examples of the acids include mineral acids such as hydrochloric acid and sulfuric acid, organic acids such as acetic acid. Examples of the salts include ammonium chloride and stannous chloride. It is also possible to use a combination of these. The amount of reducing agent to be used may be chosen appropriately in the range of 1 to 10 times mol of metal and 0.05 to 10 mol of acid and salt to the cyclic nitro compound represented by the general formula (III). The reaction temperature may be chosen in the range of 0 to 150°C, and the reaction time may not be constant depending on the reaction scale, temperature and the like but may be chosen in the range of a few min to 48 hours. Further, the reduction may be carried out by catalytic hydrogenation in the presence of a catalyst, and the catalyst may include, for example, palladium carbon. After completion of the reaction, the target compound may be isolated from the reaction mixture that contains the target compound by a standard method, and the target compound may be produced by purifying, as necessary, by recrystallization, distillation, column chromatography and the like. Also, after completion of the reaction the target compound may be used in the next reaction without isolating.

1-3) General formula (IV) → General formula (I-1)

In this reaction any inert solvent that does not strongly inhibit the proceeding of the reaction may be used. Inert solvents may include, for example: linear or cyclic chain ethers such as diethyl ether, tetrahydrofuran, and dioxane; aromatic hydrocarbons such as benzene, toluene, and xylene; halogenated hydrocarbons such as methylene chloride, chloroform, and tetrachloromethane; halogenated aromatic hydrocarbons such as chlorobenzene and dichlorobenzene; nitriles such as acetonitrile; esters such as ethyl acetate; amides such as N,N-dimethylformamide and N, N-dimethylacetamide; dimethylsulfoxide; 1,3-dimethyl-2-imidazolidinone; and water. These inert solvents may be used singly or in a mixture of two or more. Examples of bases that may be used in the present reaction include: nitrogen-containing organic bases such as triethylamine, diisopropylethylamine, 1,8-diazabicyclo[5,4,0]-undec-7-ene, and pyridine; inorganic bases such as sodium carbonate, potassium carbonate, sodium bicarbonate, sodium hydroxide, potassium hydroxide, sodium hydride, and sodium metal; organic bases such as sodium acetate and potassium acetate; and alcoholates such as sodium ethoxide and potassium t-butoxide. The amount of the base to be used may be chosen appropriately in the range of 0.5 to 5 times mol to the aniline compound represented by the general formula (IV). In this reaction a phase transfer catalyst may be used to promote the reaction. Examples of the phase transfer catalyst that may be used include: quaternary ammonium salts such as tetra-n-butyl ammonium bromide and benzyl triethyl ammonium bromide; and crown ethers such as 18-crown-6. Since the present reaction is an equal molar reaction, each reactant may be used in equal mol but any one of them may be used in excess. The reaction may be carried out at the temperature ranging from -20°C to the reflux temperature of the inert solvent used, and the reaction time may not be constant depending on the reaction scale, temperature and the like but may be chosen in the range of a few min to 48 hours. After completion of the reaction, the target compound may be isolated from the reaction mixture that contains the target compound by a standard method, and the target compound may be produced by purifying, as necessary, by recrystallization, distillation, column chromatography and the like. Also, after the completion of the reaction the target compound may be used in the next reaction without isolating.

1-4) General formula (I-1) → General formula (I)

The present reaction may be carried out according to 1-3).
After the completion of the reaction, the target compound may be isolated from the reaction mixture that contains the target compound by a standard method, and the target compound may be produced by purifying, as necessary, by recrystallization, distillation, column chromatography and the like.
The starting material represented by the general formula (II) may be produced by or according to the method described in publicly known literatures (for example, Tetrahedron Lett, 31, 4661 (1990), Synth. Commun., 24(10), 1415 (1994), and Bull. Soc. Chim. Fr., 10, 347 (1943)).

Production Process 2

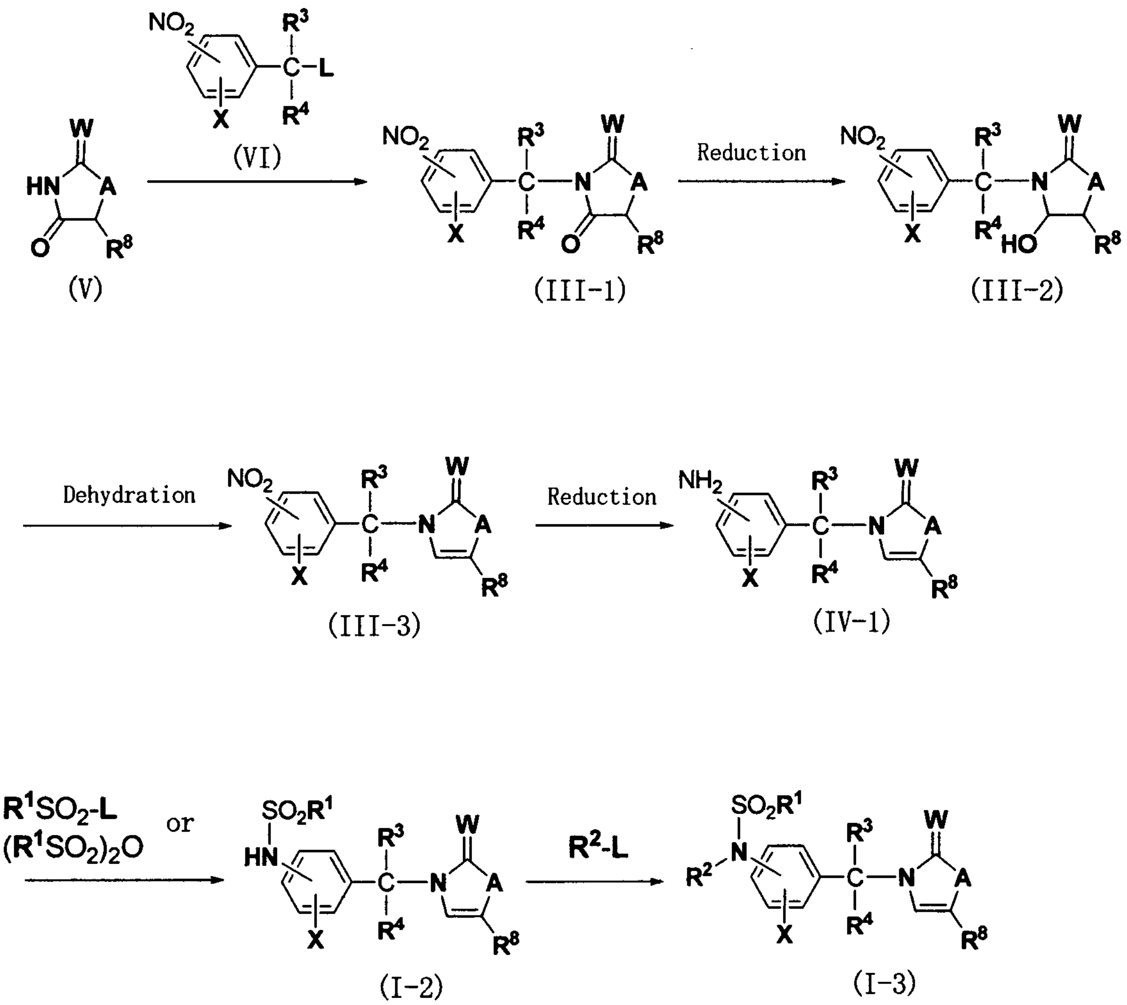


wherein $R^1$, $R^2$, $R^3$, $R^4$, $R^8$, L, A, X and W are the same as described before.

By reacting a heterocyclic compound represented by the general formula (V) with a nitrobenzene compound represented by the general formula (VI), in the presence or absence of base and inert solvent, a compound represented by the general formula (III-1) is obtained. By reducing a carbonyl group of the compound of the formula (III-1) with or without isolating it, an alcohol represented by the general formula (III-2) is obtained. With or without isolating the alcohol, this alcohol is dehydrated in the presence of acid or dehydrating agent, and in the presence or absence of an inert solvent and a cyclic unsaturated compound represented by a general formula (III-3) is obtained. With or without isolating the compound represented by (III-3), an aniline compound represented by a general formula (IV-1) is obtained by reducing the nitro group in the presence of a reducing agent, and in the presence or absence of an inert solvent. With

or without isolating this aniline compound, a haloalkyl sulfonamide derivative, which is a part of the present invention and is represented by a general formula (I-2)(when $R^6$ and $R^7$ form a double bond in the general formula (I)), may be synthesized by reacting this aniline compound with a haloalkylsulfonyl derivative, which is represented by $R^1SO_2$-L or $(R^1SO_2)_2O$, in the presence or absence of a base and an inert solvent. Further, with or without isolating the haloalkyl sulfonamide derivative represented by the general formula (I-2), a haloalkyl sulfonamide derivative of the present invention which is represented by a general formula (1-3) may be synthesized by reacting the haloalkyl sulfonamide derivative (I-2) with a compound represented by the general formula $R^2$-L.

2-1) General formula (V) → General formula (III-1)

In this reaction any inert solvent that does not strongly inhibit the proceeding of the reaction may be used. Inert solvents may include, for example: linear or cyclic chain ethers such as diethyl ether, tetrahydrofuran, and dioxane; aromatic hydrocarbons such as benzene, toluene, and xylene; amides such as dimethylformamide, N-methylacetamide, N-methylpyrrolidone, and hexamethylphosphoramide; halogenated hydrocarbons such as methylene chloride, chloroform, and tetrachloromethane; and halogenated aromatic hydrocarbons such as chlorobenzene and dichlorobenzene. These inert solvents may be used singly or in a mixture of two or more. Examples of bases that may be used in the present reaction include: nitrogen-containing organic bases such as triethylamine, diisopropylethylamine, 1,8-diazabicyclo[5,4,0]-undec-7-ene, and pyridine; inorganic bases such as sodium carbonate, potassium carbonate, sodium bicarbonate, sodium hydroxide, potassium hydroxide, sodium hydride, and sodium metal; organic bases such as sodium acetate and potassium acetate; and alcoholates such as sodium ethoxide and potassium t-butoxide. The amount of the base to be used may be chosen appropriately in the range of 0.5 to 5 times mol to the heterocyclic compound represented by the general formula (V). Since the present reaction is an equal molar reaction, each reactant may be used in equal mol but any one of them may be used in excess. The reaction may be carried out at the temperature ranging from 0 to 150˚C and the reaction time may not be constant depending on the reaction scale, temperature and the like but may be chosen in the range of a few min to 48 hours. After the completion of the reaction, the target compound may be isolated from the reaction mixture that contains the target compound by a standard method, and the target compound may be produced by purifying, as necessary, by recrystallization, distillation, column chromatography and the like. Also, after the completion of the reaction the target compound may be used in the next reaction without isolating.

2-2) General formula (III-1) → General formula (III-2)

In this reaction any inert solvent that does not strongly inhibit the proceeding of the reaction may be used. Inert solvents may include, for example: alcohols such as methanol and ethanol; ethers such as tetrahydrofuran and dioxane; halogenated hydrocarbons such as methylene chloride, chloroform, and tetrachloromethane; and water. These inert solvents may be used singly or in a mixture of two or more. Reducing agents that may be used in the present reaction include metallic hydride complex compound such as sodium borohydride, lithium borohydride, zinc borohydride, lithium aluminum hydride, and diisobutylaluminum hydride. The amount of reducing agent to be used may be chosen appropriately in the range of 0.25 to 10 times mol of the compound represented by the general formula (III-1). The reaction may be carried out at the temperature ranging from 0 to 150˚C and the reaction time may not be constant depending on the reaction scale, temperature and the like but may be chosen in the range of a few min to 48 hours. After the completion of the reaction, the target compound may be isolated from the reaction mixture that contains the target compound by a standard method, and the target compound may be produced by purifying, as necessary, by recrystallization, distillation, column chromatography and the like. Also, after the completion of the reaction the target compound may be used in the next reaction without isolating.

2-3) General formula (III-2) → General formula (III-3)

In this reaction any inert solvent that does not strongly inhibit the proceeding of the reaction may be used. Inert solvents may include, for example: linear or cyclic chain ethers such as diethyl ether, tetrahydrofuran, and dioxane; aromatic hydrocarbons such as benzene, toluene, and xylene; halogenated hydrocarbons such as methylene chloride, chloroform, and tetrachloromethane; halogenated aromatic hydrocarbons such as chlorobenzene and dichlorobenzene. These inert solvents may be used singly or in a mixture of two or more. Also, in the case where a dehydrating agent is used, the inert solvents such as pyridine, dimethylformamide, N-methylacetamide, N-methylpyrrolidone, and hexamethylphosphoramide may be used. Acids that may be used in the present reaction include, for example: metallic acids such as hydrochloric acid and sulfuric acid; organic acids such as formic acid, acetic acid, and trifluoroacetic acid; and sulfonic acids such as methanesulfonic acid, trifluoromethanesulfonic acid, and p-toluenesulfonic acid. The amount of acid to be used may be chosen appropriately in the range from 0.1 to 10 times mol of alcohol represented by the general formula (III-2). Dehydrating agents that may be used in the present reaction include, for example: halogenating agents

such as thionyl chloride and phosphoryl chloride; sulfonating agent such as methanesulfonyl chloride; and esterifying agents such as acetic anhydride and phthalic anhydride. The amount of dehydrating agent to be used may be chosen appropriately in the range of 1 to 10 times mol of the alcohols represented by the general formula (111-2). The reaction may be carried out at the temperature ranging from 0 to 150˚C, and the reaction time may not be constant depending on the reaction scale, temperature and the like but may be chosen in the range of a few min to 48 hours. After the completion the reaction, the target compound may be isolated from the reaction mixture that contains the target compound by a standard method, and the target compound may be produced by purifying, as necessary, by recrystallization, distillation, column chromatography and the like. Also, after the reaction the target compound may be used in the next reaction without isolating.

2-4) General formula (III-3) → General formula (IV-1)

The present reaction may be carried out similarly to 1-2).

2-5) General formula (IV-1) → General formula (1-2)

The present reaction may be carried out similarly to 1-3).

2-6) General formula (I-2) → General formula (I-3)

The present reaction may be carried out similarly to 1-4).
The heterocyclic compound represented by the general formula (V) may be produced by or according to the method described in the publicly known literatures (for example, J. Am. Chem. Soc, 67, 522-523 (1945), JP-A-58-183603).

Production Process 3

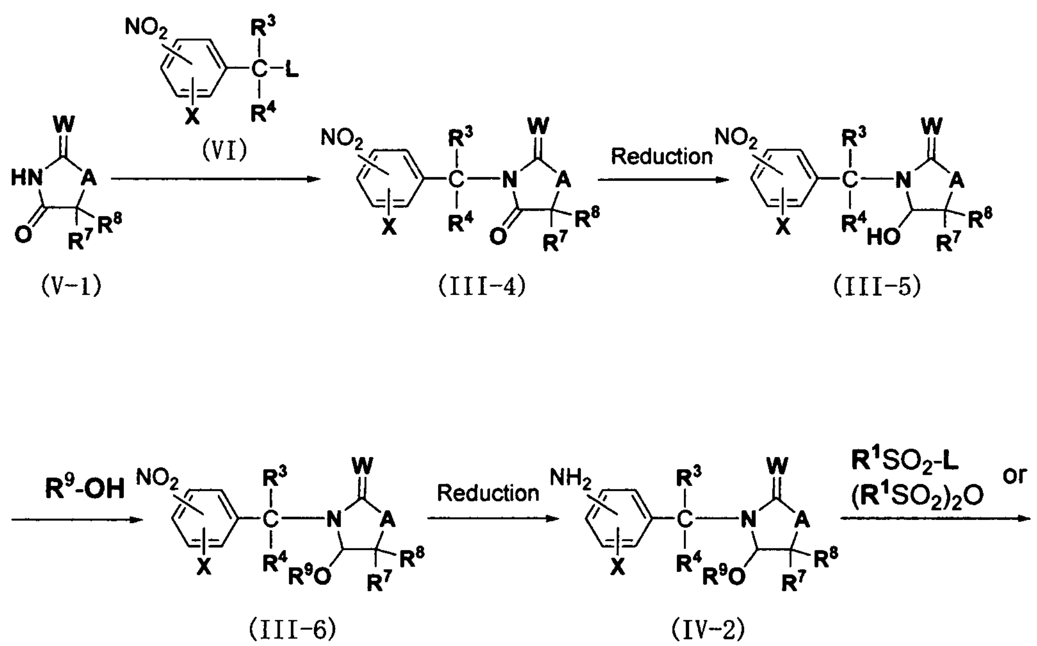

(V-1) (III-4) (III-5)

(III-6) (IV-2)

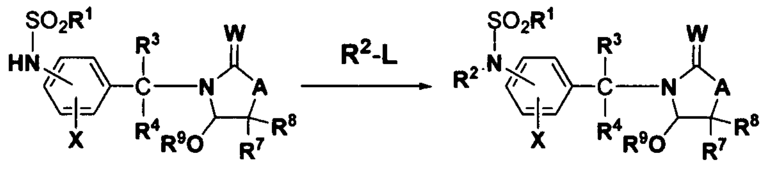

(I-4) (I-5)

wherein $R^1$, $R^2$, $R^3$, $R^4$, $R^7$, $R^8$, L, A, X and W are the same as described before and $R^9$ is ($C_1$-$C_6$) alkyl group.

By reacting a heterocyclic compound represented by the general formula (V-1) with a nitrobenzene compound represented by the general formula (VI), in the presence or absence of a base and an inert solvent, a compound represented by the general formula (III-4) is obtained. By reducing a carbonyl group of the compound of the formula (III-4) with or without isolating it, an alcohol represented by the general formula (III-5) is obtained. With or without isolating the alcohol, this alcohol is reacted with an alcohol represented by the formula $R^9OH$ in the presence of acid, and an ether represented by a general formula (111-6) is obtained. By reducing the nitro group of the ether, which may be isolated or not, in the presence of a reducing agent, and in the presence or absence of an inert solvent, an aniline compound represented by a general formula (IV-2) is obtained. With or without isolating this aniline compound, a haloalkyl sulfonamide derivative, which is a part of the present invention and is represented by a general formula (I-4) (when $R^5$ or $R^6$ is alkoxy group in the general formula (I)), may be synthesized by reacting this aniline compound with a haloalkylsulfonyl derivative, which is represented by $R^1SO_2$-L or $(R^1SO_2)_2O$, in the presence or absence of a base and an inert solvent. Further, with or without isolating the haloalkyl sulfonamide derivative represented by the general formula (I-4), a haloalkyl sulfonamide derivative of the present invention which is represented by a general formula (I-5) may be produced by reacting the haloalkyl sulfonamide derivative (I-2) with a compound represented by the general formula $R^2$-L.

3-1) General formula (V-1) → General formula (III-4)

The present reaction may be carried out similarly to 2-1).

3-2) General formula (III-4) → General formula (III-5)

The present reaction may be carried out similarly to 2-2).

3-3) General formula (III-5) → General formula (III-6)

The solvent used in the present reaction is an alcohol corresponding to $R^9$ (for example, methanol, ethanol, n-propanol, isopropyl alcohol, n-butanol, isobutyl alcohol, sec-butyl alcohol, or t-butyl alcohol). Acids that may be used in the present reaction include, for example: mineral acids such as hydrochloric acid and sulfuric acid; organic acids such as formic acid, acetic acid, and trifluoroacetic acid; and sulfonic acids such as methanesulfonic acid, trifluoromethanesulfonic acid, and p-toluenesulfonic acid. The amount of acid to be used may be chosen appropriately in the range 0.01 to 10 times mol to the alcohol represented by the general formula (III-5). The reaction temperature may be chosen in the range 0 to 150˚C, and the reaction time may be chosen appropriately in the range of a few min to 48 hours although it may not be constant depending on the reaction scale and temperature. After the completion the reaction, the target compound may be isolated from the reaction mixture that contains the target compound by a standard method, and the target compound may be produced by purifying, as necessary, by recrystallization, distillation, column chromatography and the like. Also, after the completion the reaction the target compound may be used in the next reaction without isolating.

3-4) General formula (III-6) → General formula (IV-2)

The present reaction may be carried out similarly to 1-2).

3-5) General formula (IV-2) → General formula (I-4)

The present reaction may be carried out similarly to 1-3).

3-6) General formula (1-4) → General formula (1-5)

The present reaction may be carried out similarly to 1-4).
The heterocyclic compound represented by the general formula (V-1) may be produced by or according to the method described in the publicly known literatures (for example, J. Am. Chem. Soc, 67, 522-523 (1945), JP-A-58-183603).

Production Process 4

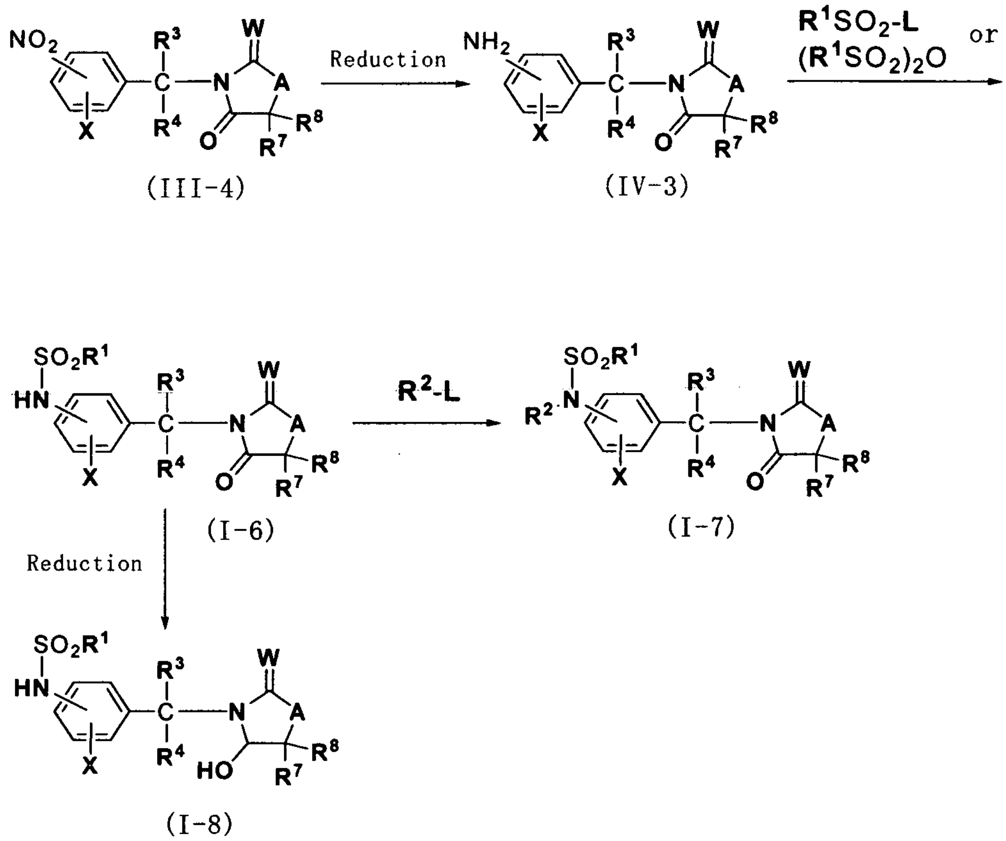


(III-4) (IV-3)

(I-6) (I-7)

(I-8)

wherein $R^1$, $R^2$, $R^3$, $R^4$, $R^7$, $R^8$, L, A, X and W are the same as described before.

By reducing the nitro group of a compound represented by the general formula (III-4) in the presence of a reducing agent and in the presence or absence of an inert solvent, an aniline compound represented by the general formula (IV-3) is obtained. With or without isolating this aniline compound, a haloalkyl sulfonamide derivative, which is a part of the compound of the present invention and is represented by a general formula (I-6) (when $R^5$ and $R^6$ form carbonyl group in the general formula (I)), may be produced by reacting this aniline compound with a haloalkylsulfonyl derivative, which is represented by $R^1SO_2$-L or $(R^1SO_2)_2O$, in the presence or absence of a base and an inert solvent. Further, with or without isolating the haloalkyl sulfonamide derivative represented by the general formula (I-6), a haloalkyl sulfonamide derivative of the present invention which is represented by a general formula (1-7) may be produced by reacting the derivative with a compound represented by the general formula $R^2$-L. Alternatively, with or without isolating the haloalkyl sulfonamide derivative represented by the general formula (I-6), a haloalkyl sulfonamide derivative of the present invention which is represented by a general formula (I-8) may be produced by reducing the carbonyl group in the presence of a reducing agent, and in the presence or absence of an inert solvent.

4-1) General formula (III-4) → General formula (IV-3)

The present reaction may be carried out similarly to 1-2).

4-2) General formula (IV-3) → General formula (I-6)

The present reaction may be carried out similarly to 1-3).

4-3) General formula (1-6) → General formula (I-7)

The present reaction may be carried out similarly to 1-4).

4-4) General formula (I-6) → General formula (1-8)

The present reaction may be carried out similarly to 2-2)

Production Process 5

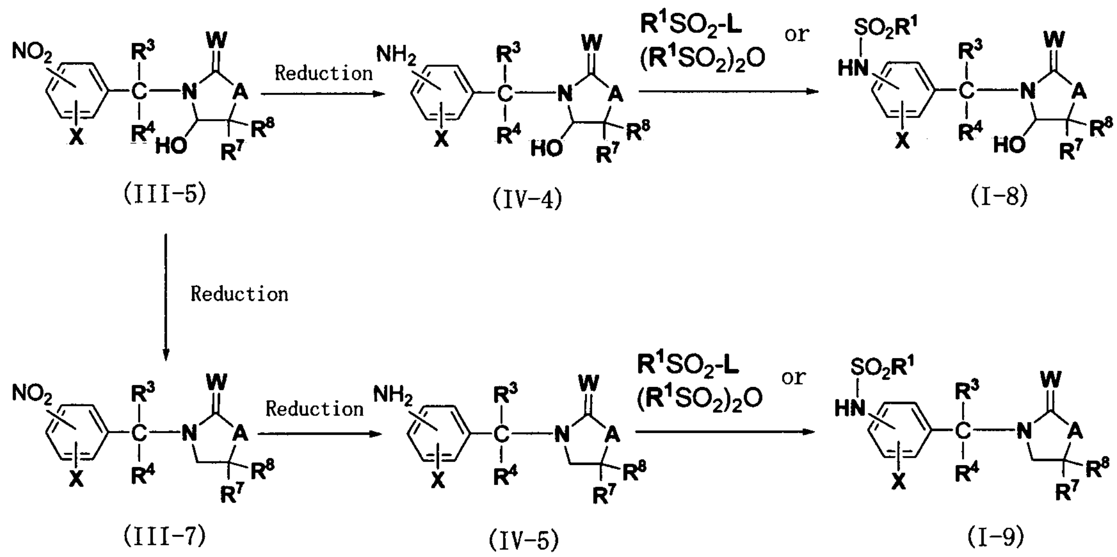


(III-5) (IV-4) (I-8)

(III-7) (IV-5) (I-9)

wherein $R^1$, $R^3$, $R^4$, $R^7$, $R^8$, L, A, X and W are the same as described before.

An aniline compound represented by the general formula (IV-4) is obtained by reducing the nitro group of an alcohol represented by the general formula (III-5) in the presence of a reducing agent and in the presence or absence of an inert solvent. A haloalkyl sulfonamide derivative represented by the general formula (I-8) of the present invention may be produced by reacting the aniline compound (IV-4), with or without isolating it, with a haloalkylsulfonyl derivative represented by the formula $R^1SO_2$-L or $(R^1SO_2)_2O$ in the presence or absence of a base and an inert solvent.
Also, by reducing the hydroxyl group of an alcohol represented by the general formula (111-5) in the presence of acid and triethyl silane, in the presence or absence of inert solvent, a saturated heterocyclic ring compound represented with general formula (III-7) is obtained. By reducing the nitro group of this saturated heterocyclic ring compound (III-7), with or without isolating it, in the presence of reducing agent, and in the presence or absence of an inert solvent, an aniline compound represented by the general formula (IV-5) is obtained. A haloalkyl sulfonamide derivative represented by the general formula (I-8) of the present invention may also be produced by reacting the aniline compound (IV-5), with or without isolating it, with a haloalkylsulfonyl derivative represented by the formula $R^1SO_2$-L or $(R^1SO_2)_2O$ in the presence or absence of a base and an inert solvent.

5-1) General formula (III-4) → General formula (IV-4)

The present reaction may be carried out similarly to 1-2).

5-2) General formula (IV-4) → General formula (I-8)

The present reaction may be carried out similarly to 1-3).

5-3) General formula (III-5) → General formula (III-7)

The amount of triethyl silane used in the present reaction may be chosen in the range of 0.5 to 10 times of mol to the alcohol compound represented in the general formula (III-5), preferably in the range of 1 to 3 times of mol. In this reaction any inert solvent that does not strongly inhibit the proceeding of the reaction may be used. Inert solvent may include, for example: linear or cyclic chain ethers such as diethyl ether, tetrahydrofuran, and dioxane; aromatic hydrocarbons such as benzene, toluene, and xylene; halogenated hydrocarbons such as methylene chloride, chloroform, and tetrachloromethane; and halogenated aromatic hydrocarbons such as chlorobenzene and dichlorobenzene. These inert solvents may be used singly or in a mixture of two or more. Acids that may be used in the present reaction include, for example: mineral acids such as hydrochloric acid and sulfuric acid; organic acids such as formic acid, acetic acid, and trifluoroacetic acid; and sulfonic acids such as methanesulfonic acid, trifluoromethanesulfonic acid, and p-toluenesulfonic acid. The amount of the acid to be used may be chosen appropriately in the range of 0.1 to 50 times of mol to the alcohol compound represented by the general formula (III-5). The reaction temperature may be chosen in the range of 0˚C to the boiling point of the solvent used and the reaction time may not be constant depending on the reaction scale, temperature and the like but may be chosen in the range of a few min to 48 hours. After the completion of the reaction, the target compound may be isolated from the reaction mixture that contains the target compound by a standard method, and the target compound may be produced by purifying, as necessary, by recrystallization, distillation, column chromatography and the like. Also, after the completion of the reaction the target compound may be used in the next reaction without isolating.

5-4) General formula (III-7) → General formula (IV-5)

The present reaction may be carried out similarly to 1-2).

5-5) General formula (IV-5) → General formula (I-9)

The present reaction may be carried out similarly to 1-3).
In the following Tables 1 and 2, examples of haloalkylsulfonanilide derivatives represented by the general formula (I) of the present invention are shown, and examples of the intermediates, aniline compounds represented by the general formula (IV), are shown in Tables 3 and 4. However, the present invention is not limited to these. In the tables, "Me" is methyl group, "Et" is ethyl group, "Pr" is propyl group, "Bu" is butyl group, "Hex" is hexyl group, "Ac" is acetyl group, "Ph" is phenyl group, "n-" is normal, "i-" is iso, "s-" is secondary, "t-" is tertiary, and "c-" is cycloaliphatic hydrocarbon group. "-" indicates $R^6$ and $R^7$ are bound and forms a double bond in the ring. "Q1" and "Q2" represent the structures shown below.

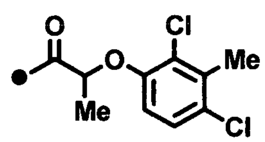

Q1

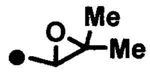

Q2

"(R form)" and "(S form)" show the stereochemistry of the asymmetrical carbon to which each substituent connects. "Position of the substituent" represents the position of haloalkylsulfonylamino group, nitro group or amino group on the benzene ring in each structural formula, "properties" represent melting point (˚C) or refractive index $n_D$ (˚C). Table 5 shows $^1$H-NMR spectra of the compounds, the properties of which are recorded in Table 1 to Table 4 as NMR.
[Table 1]

General formula (I)

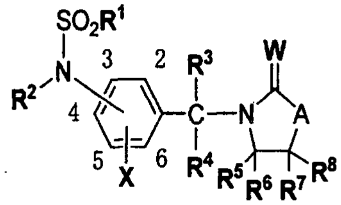

(I)

Table 1 ($X=R^3=R^4=H$)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-1 | 2 | $CF_3$ | H | H | H | H | H | O | O | 140-143 |
| 1-2 | 2 | $CF_3$ | $CO_2$-i-Bu | H | H | H | H | O | O | 1.4888(23) |
| 1-3 | 2 | $CF_3$ | H | Me | H | H | H | O | O | 1.4911(20) |
| 1-4 | 2 | $CF_3$ | $CO_2$+Bu | Me | H | H | H | O | O | 1.4802(20) |
| 1-5 | 2 | $CF_3$ | H | Et | H | H | H | O | O | 81.4-83.6 |
| 1-6 | 2 | $CF_3$ | $CO_2$ | Et | H | H | H | O | O | 69.3-71.7 |
| 1-7 | 2 | $CF_3$ | H | n-Pr | H | H | H | O | O | 98.7-99.0 |
| 1-8 | 2 | $CF_3$ | $CO_2$-i-Bu | n-Pr | H | H | H | O | O | 1.4858(22) |
| 1-9 | 2 | $CF_3$ | H | i-Pr | H | H | H | O | O | 132.5 |
| 1-10 | 2 | $CF_3$ | $CO_2$+Bu | i-Pr | H | H | H | O | O | 1.4861(22) |
| 1-11 | 2 | $CF_3$ | H | n-Bu | H | H | H | O | O | 93.2 |
| 1-12 | 2 | $CF_3$ | $CO_2$+Bu | n-Bu | H | H | H | O | O | 1.4885(20) |
| 1-13 | 2 | $CF_3$ | H | $CO_2Me$ | H | H | H | O | O | 1.4915(21) |
| 1-14 | 2 | $CF_3$ | $CO_2$-i-Bu | $CO_2Me$ | H | H | H | O | O | 1.4812(23) |
| 1-15 | 2 | $CF_3$ | H | Me | Me | H | H | O | O | 134.9 |
| 1-16 | 2 | $CF_3$ | $CO_2$-i-Bu | Me | Me | H | H | O | O | 65.0-65.8 |
| 1-17 | 2 | $CF_3$ | H | H | H | Me | H | O | O | 1.4988(24) |
| 1-18 | 2 | $CF_3$ | $CO_2$-i-Bu | H | H | Me | H | O | O | 1.4738(25) |
| 1-19 | 2 | $CF_3$ | H | H | H | $CF_3$ | H | O | O | 106.4-107.0 |
| 1-20 | 2 | $CF_3$ | $CO_2$-i-Bu | H | H | $CF_3$ | H | O | O | 107.8-112.1 |

(continued)

Table 1 ($X=R^3=R^4=H$)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-21 | 2 | $CF_3$ | H | H | H | Me | H | O | S | 74-76 |
| 1-22 | 2 | $CF_3$ | $CO_2$-i-Bu | H | H | Me | H | O | S | 1.5168(22) |
| 1-23 | 2 | $CF_3$ | H | H | H | Et | H | O | O | 1.4885(23) |
| 1-24 | 2 | $CF_3$ | $CO_2$+Bu | H | H | Et | H | O | O | 85-87 |
| 1-25 | 3 | $CF_3$ | H | H | H | Et | H | O | O | 1.4889(20) |
| 1-26 | 3 | $CF_3$ | $CO_2$-i-Bu | H | H | Et | H | O | O | 1.4752(22) |
| 1-27 | 4 | $CF_3$ | H | H | H | Et | H | O | O | 81.5 |
| 1-28 | 4 | $CF_3$ | $CO_2$ | H | H | Et | H | O | O | 1.4854(23) |
| 1-29 | 2 | $CF_3$ | H | H | H | n-Pr | H | O | O | 1.4910(21) |
| 1-30 | 2 | $CF_3$ | $CO_2$ | H | H | n-Pr | H | O | O | 83.8 |
| 1-31 | 2 | $CF_3$ | H | H | H | i-Pr | H | O | O | 70.0-73.9 |
| 1-32 | 2 | $CF_3$ | $CO_2$-i-Bu | H | H | i-Pr | H | O | O | 98.4-99.1 |
| 1-33 | 2 | $CF_3$ | H | H | H | t-Bu | H | O | O | 67.0-70.6 |
| 1-34 | 2 | $CF_3$ | $CO_2$-i-Bu | H | H | t-Bu | H | O | O | 69.2-73.3 |
| 1-35 | 2 | $CF_3$ | H | H | H | $CH_2OMe$ | H | O | O | 1.4968(23) |
| 1-36 | 2 | $CF_3$ | $CO_2$+Bu | H | H | $CH_2OMe$ | H | O | O | 70.9-75.1 |
| 1-37 | 2 | $CF_3$ | H | H | H | $CH_2OAc$ | H | O | O | 1.4875(23) |
| 1-38 | 2 | $CF_3$ | $CO_2$-i-Bu | H | H | $CH_2OAc$ | H | O | O | 91 |
| 1-39 | 2 | $CF_3$ | H | H | H | $CH_2Cl$ | H | O | O | 1.5068(22) |
| 1-40 | 2 | $CF_3$ | $CO_2$-i-Bu | H | H | $CH_2Cl$ | H | O | O | 1.4964(22) |
| 1-41 | 2 | $CF_3$ | H | H | H | Ph | H | O | O | 1.5304(24) |
| 1-42 | 2 | $CF_3$ | $CO_2$-i-Bu | H | H | Ph | H | O | O | 1.5002(25) |
| 1-43 | 2 | $CF_3$ | H | H | H | Me | Me | O | O | 1.4822(23) |
| 1-44 | 2 | $CF_3$ | $CO_2$-i-Bu | H | H | Me | Me | O | O | 59-61 |
| 1-45 | 2 | $CF_3$ | H | H | H | $(CH_2)_5$ | | O | O | 104.4 |
| 1-46 | 2 | $CF_3$ | $CO_2$ | H | H | $(CH_2)_5$ | | O | O | 90.3 |
| 1-47 | 2 | $CF_3$ | H | H | $(CH_2)_4$ | | H | O | O | 114.6-115.7 |
| 1-48 | 2 | $CF_3$ | $CO_2$+Bu | H | $(CH_2)_4$ | | H | O | O | 1.4976(23) |
| 1-49 | 2 | $CF_3$ | H | H | H | Me | Me | O | O | 1.5258(18) |
| 1-50 | 2 | $CF_3$ | $CO_2$-i-Bu | H | H | Me | Me | O | O | 117.9 |
| 1-51 | 2 | $CF_3$ | H | H | H | $CH_2OPh$ | H | O | O | 1.5249(18) |

(continued)

Table 1 ($X=R^3=R^4=H$)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-52 | 2 | $CF_3$ | $CO_2$-i-Bu | H | H | $CH_2OPh$ | H | O | O | 131 |
| 1-53 | 2 | $CF_3$ | CO-i-Bu | H | H | Et | H | O | O | 1.4946(23) |
| 1-54 | 2 | $CF_3$ | $CO_2Me$ | H | H | Me | H | O | O | 1.4978(22) |
| 1-55 | 2 | $CF_3$ | H | H | H | Et(S form) | H | O | O | 82.9-84.6 |
| 1-56 | 2 | $CF_3$ | $CO_2$-i-Bu | H | H | Et(S form) | H | O | O | 77.0-79.6 |
| 1-57 | 2 | $CF_3$ | H | H | H | Et(R form) | H | O | O | 83.5-83.7 |
| 1-58 | 2 | $CF_3$ | $CO_2$+Bu | H | H | Et(R form) | H | O | O | 78.9-82.0 |
| 1-59 | 2 | $CF_3$ | H | H | H | Et | Me | O | O | 1.4862(22) |
| 1-60 | 2 | $CF_3$ | $CO_2$+Bu | H | H | Et | Me | O | O | 1.4848(23) |
| 1-61 | 2 | $CF_3$ | H | H | H | Et | Et | O | O | 1.4858(23) |
| 1-62 | 2 | $CF_3$ | $CO_2$+Bu | H | H | Et | Et | O | O | 1.4842(23) |
| 1-63 | 2 | $CF_3$ | $CO_2$-n-Bu | H | H | Me | Me | O | O | 89.3-93.7 |
| 1-64 | 2 | $CF_3$ | $CO_2Me$ | H | H | Me | Me | O | O | 1.4816(23) |
| 1-65 | 2 | $CF_3$ | $CO_2CH_2CCl_3$ | H | H | Me | Me | O | 0 | 1.4948(23) |
| 1-66 | 2 | $CF_3$ | $CO_2CH_2C(CH_3)_3$ | H | H | Me | Me | O | O | 1.4768(23) |
| 1-67 | 2 | $CF_3$ | $CO_2CH_2CH(CH_3)\ C_2H_5$ | H | H | Me | Me | O | O | 1.4782(23) |
| 1-68 | 2 | $CF_3$ | $CO_2CH_2CH(C_2H_5)\ (CH_2)_3CH_3$ | H | H | Me | Me | 0 | O | 74.1-75.0 |
| 1-69 | 2 | $CF_3$ | H | H | H | t-Bu | Me | 0 | O | 122.7-125.4 |
| 1-70 | 2 | $CF_3$ | $CO_2$-i-Bu | H | H | t-Bu | Me | O | O | 1.4958(23) |
| 1-71 | 2 | $CF_3$ | H | H | H | c-Pr | Me | O | O | 1.4958(23) |
| 1-72 | 2 | $CF_3$ | $CO_2$-i-Bu | H | H | c-Pr | Me | O | O | 58.0-60.9 |
| 1-73 | 2 | $CF_3$ | H | Me | Me | Me | Me | 0 | O | 102.3-106.6 |
| 1-74 | 2 | $CF_3$ | $CO_2$-i-Bu | Me | Me | Me | Me | O | O | 1.4818(24) |
| 1-75 | 2 | $CF_3$ | $CO_2$-n-Pr | H | H | Me | Me | O | O | 72.5-73.6 |
| 1-76 | 2 | $CF_3$ | $CO_2$-i-Pr | H | H | Me | Me | 0 | O | 102.8-105.6 |
| 1-77 | 2 | $CF_3$ | $CO_2CH_2CH\ (C_2H_5)_2$ | H | H | Me | Me | 0 | O | 782 |
| 1-78 | 2 | $CF_3$ | $CO_2C_2H_4O$-i-Pr | H | H | Me | Me | 0 | O | 73.3 |
| 1-79 | 2 | $CF_3$ | $CO_2CH_2C{\equiv}CH$ | H | H | Me | Me | O | 0 | 1.4878(23) |
| 1-80 | 2 | $CF_3$ | $CO_2Et$ | H | H | Me | Me | O | 0 | 82.3-84.5 |
| 1-81 | 2 | $CF_3$ | $CO_2C_2H_4OCH_3$ | H | H | Me | Me | O | 0 | 112.0-113.8 |
| 1-82 | 2 | $CF_3$ | $CO_2CH_2CH{=}CH_2$ | H | H | Me | Me | O | O | 88.8-91.0 |

(continued)

| Table 1 ($X=R^3=R^4=H$) | | | | | | | | | | |
|---|---|---|---|---|---|---|---|---|---|---|
| Compound No. | Position of the substituent | $R^1$ | $R^2$ | $R^5$ | $R^6$ | $R^7$ | $R^8$ | A | W | Properties |
| 1-83 | 2 | $CF_3$ | $CO_2Bn$ | H | H | Me | Me | O | O | 158.4-1592 |
| 1-84 | 2 | $CF_3$ | $CO_2(CH_2)_{15}CH_3$ | H | H | Me | Me | O | O | 66.0-67.4 |
| 1-85 | 2 | $CF_3$ | $CO_2Ph$ | H | H | Me | Me | O | O | 128.2-129.8 |
| 1-86 | 2 | $CF_3$ | $CO_2$-i-Bu | H | H | $(CH_2)_3$ | | O | O | 73.7-74.8 |
| 1-87 | 2 | $CF_3$ | H | H | H | $(CH_2)_3$ | | O | O | 1.5020(25) |
| 1-88 | 2 | $CF_3$ | H | H | H | $(CH_2)_2$ | | O | O | 1.5018(24) |
| 1-89 | 2 | $CF_3$ | $CO_2$-i-Bu | H | H | $(CH_2)_2$ | | O | O | 76.7-80.1 |
| 1-90 | 2 | $CF_3$ | $CO_2$-n-Pr | H | H | Et | Me | O | O | 59.4-61.3 |
| 1-91 | 2 | $CF_3$ | $CO_2(CH_2)_4CH_3$ | H | H | Et | Me | O | O | 1.4840(22) |
| 1-92 | 2 | $CF_3$ | $CO_2(CH_2)_4CH_3$ | H | H | Me | Me | O | O | 74.9-75.8 |
| 1-93 | 2 | $CF_3$ | $CO_2(CH_2)_5CH_3$ | H | H | Me | Me | O | O | 52.7-52.9 |
| 1-94 | 2 | $CF_3$ | $CO_2(CH_2)_6CH_3$ | H | H | Me | Me | O | O | 42.4-52.4 |
| 1-95 | 2 | $CF_3$ | $CO_2$-i-Bu | H | Me | Me | Me | O | O | 55.6-58.8 |
| 1-96 | 2 | $CF_3$ | H | H | Me | Me | Me | O | O | 1.4892(21) |
| 1-97 | 2 | $CF_3$ | $CO_2Et$ | H | H | Et | Et | O | O | 42.1-49.7 |
| 1-98 | 2 | $CF_3$ | $CO_2$-n-Pr | H | H | Et | Et | O | O | 41.8-49.0 |
| 1-99 | 2 | $CF_3$ | $CO_2$-n-Bu | H | H | Et | Et | O | O | 1.4750(23) |
| 1-100 | 2 | $CF_3$ | $CO_2CH_2C(CH_3)_3$ | H | H | Et | Et | O | O | 1.4722(23) |
| 1-101 | 2 | $CF_3$ | H | H | H | i-Pr | Me | O | O | 85.1-89.0 |
| 1-102 | 2 | $CF_3$ | $CO_2$ | H | H | i-Pr | Me | O | O | 1.4754(22) |
| 1-103 | 2 | $CF_3$ | H | H | H | i-Bu | Me | O | O | 1.4874(20) |
| 1-104 | 2 | $CF_3$ | $CO_2$-i-Bu | H | H | i-Bu | Me | O | O | 1.4744(21) |
| 1-105 | 2 | $CF_3$ | $CO_2CH_2C(CH_3)_3$ | H | H | Et | Me | O | O | 58.7-63.4 |
| 1-106 | 2 | $CF_3$ | H | H | H | $CH_2NHCO_2$ -t-Bu | H | O | O | NMR |
| 1-107 | 2 | $CF_3$ | $CO_2$-i-Bu | H | H | $CH_2NHCO_2$ -t-Bu | H | O | O | 1.4806(24) |
| 1-108 | 2 | $CF_3$ | $CO_2Me$ | H | H | Et | Me | O | O | 1.4836(25) |
| 1-109 | 2 | $CF_3$ | $CO_2Me$ | H | H | Et | Et | O | O | 1.4816(25) |
| 1-110 | 2 | $CF_3$ | $CO_2Me$ | H | H | c-Pr | Me | O | O | 106.7-108.3 |
| 1-111 | 2 | $CF_3$ | $CO_2Et$ | H | H | c-Pr | Me | O | O | 82.9-842 |
| 1-112 | 2 | $CF_3$ | $CO_2$-n-Pr | H | H | c-Pr | Me | O | O | 87.5-89.4 |
| 1-113 | 2 | $CF_3$ | $CO_2$-n-Bu | H | H | c-Pr | Me | O | O | 1.4908(26) |

(continued)

Table 1 ($X=R^3=R^4=H$)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-114 | 2 | $CF_3$ | $CO_2Me$ | H | H | i-Pr | Me | O | O | 1.4882(26) |
| 1-115 | 2 | $CF_3$ | $CO_2Et$ | H | H | i-Pr | Me | O | O | 73.5-74.7 |
| 1-116 | 2 | $CF_3$ | $CO_2$-n-Pr | H | H | i-Pr | Me | O | O | 75.7-76.9 |
| 1-117 | 2 | $CF_3$ | $CO_2$-n-Bu | H | H | i-Pr | Me | O | O | 1.4814(26) |
| 1-118 | 2 | $CF_3$ | $CO_2$-i-Pr | H | H | c-Pr | Me | O | O | 1.4838(26) |
| 1-119 | 2 | $CF_3$ | $CO_2C_2H_4OCH_3$ | H | H | c-Pr | Me | O | O | 1.4878(26) |
| 1-120 | 2 | $CF_3$ | H | H | H | n-Pr | Me | O | | 1.4820(26) |
| 1-121 | 2 | $CF_3$ | H | H | H | n-Bu | Me | O | O | 1.4870(26) |
| 1-122 | 2 | $CF_3$ | $CO_2$-i-Bu | H | H | n-Pr | Me | O | O | 1.4812(26) |
| 1-123 | 2 | $CF_3$ | $CO_2$-i-Bu | H | H | n-Bu | Me | O | O | 1.4800(26) |
| 1-124 | 2 | $CF_3$ | $CO_2Me$ | H | H | i-Bu | Me | O | O | 1.4834(26) |
| 1-125 | 2 | $CF_3$ | $CO_2Et$ | H | H | i-Bu | Me | O | O | 1.4806(26) |
| 1-126 | 2 | $CF_3$ | $CO_2Me$ | H | H | n-Pr | Me | O | O | 1.4816(25) |
| 1-127 | 2 | $CF_3$ | $CO_2Et$ | H | H | n-Pr | Me | O | O | 1.4792(25) |
| 1-128 | 2 | $CF_3$ | $CO_2Me$ | H | H | n-Bu | Me | O | O | 1.4812(25) |
| 1-129 | 2 | $CF_3$ | $CO_2Et$ | H | H | n-Bu | Me | O | O | 1.4788(25) |
| 1-130 | 2 | $CF_3$ | H | H | H | n-Pr | Et | O | O | 1.4900(26) |
| 1-131 | 2 | $CF_3$ | H | H | H | i-Pr | Et | O | O | 1.4834(26) |
| 1-132 | 2 | $CF_3$ | $CO_2$+Bu | H | H | n-Pr | Et | O | O | 1.4774(25) |
| 1-133 | 2 | $CF_3$ | $CO_2$+Bu | H | H | i-Pr | Et | O | O | 1.4772(25) |
| 1-134 | 2 | $CF_3$ | $CO_2C_2H_4OCH_3$ | H | H | Et | Et | O | O | 49-51 |
| 1-135 | 2 | $CF_3$ | H | H | H | $CF_3$ | Me | O | O | 1.4652(27) |
| 1-136 | 2 | $CF_3$ | $CO_2$-i-Bu | H | H | $CF_3$ | Me | O | O | 100.5-1024 |
| 1-137 | 2 | $CF_3$ | H | H | H | $CH_2OMe$ | Me | O | O | 1.4840(25) |
| 1-138 | 2 | $CF_3$ | $CO_2$+Bu | H | H | $CH_2OMe$ | Me | O | O | 1.4760(23) |
| 1-139 | 2 | $CF_3$ | Q1 | H | H | Me | Me | O | O | 1.5301(18) |
| 1-140 | 2 | $CF_3$ | H | H | H | Ph | Me | O | O | 1.5260(19) |
| 1-141 | 2 | $CF_3$ | $CO_2$+Bu | H | H | Ph | Me | O | O | 1.5088(19) |
| 1-142 | 2 | $CF_3$ | $CO_2Me$ | H | H | Ph | Me | O | O | 1.4782(22) |
| 1-143 | 2 | $CF_3$ | $CO_2Me$ | H | H | $CF_3$ | Me | O | O | 1.4650(22) |
| 1-144 | 2 | $CF_3$ | $CO_2Me$ | H | H | n-Pr | Et | O | O | 1.4838(22) |

(continued)

| Table 1 ($X=R^3=R^4=H$) | | | | | | | | | | |
|---|---|---|---|---|---|---|---|---|---|---|
| Compound No. | Position of the substituent | $R^1$ | $R^2$ | $R^5$ | $R^6$ | $R^7$ | $R^8$ | A | W | Properties |
| 1-145 | 2 | $CF_3$ | $CO_2Me$ | H | H | i-Pr | Et | O | O | 1.4882(22) |
| 1-146 | 2 | $CF_3$ | $CO_2CH_2C(CH_3)_3$ | H | H | $CH_2OMe$ | Me | O | O | 1.4798(22) |
| 1-147 | 2 | $CF_3$ | $CO_2Me$ | H | H | $CH_2OMe$ | Me | O | O | 1.4874(22) |
| 1-148 | 2 | $CF_3$ | $CO_2Me$ | H | Me | Me | Me | O | O | 138.6-145.0 |
| 1-149 | 2 | $CF_3$ | $CO_2Me$ | H | H | $(CH_2)_5$ | | O | O | 1.4978(22) |
| 1-150 | 2 | $CF_3$ | $CO_2Me$ | Me | H | H | H | O | O | 1.4902(22) |
| 1-151 | 2 | $CF_3$ | $CO_2Me$ | H | H | $CF_3$ | H | O | O | 91.6-99.6 |
| 1-152 | 2 | $CF_3$ | $CO_2Me$ | H | | $(CH_2)_4$ | H | O | O | 1.3768(22) |
| 1-153 | 2 | $CF_3$ | $CO_2Me$ | H | H | t-Bu | Me | O | O | 109.0-112.9 |
| 1-154 | 2 | $CF_3$ | H | H | H | c-Hex | Me | O | O | 1.5024(22) |
| 1-155 | 2 | $CF_3$ | H | H | H | $CH_2OPh$ | Me | O | O | 1.5198(22) |
| 1-156 | 2 | $CF_3$ | $CO_2$-i-Bu | H | H | c-Hex | Me | O | O | 1.4934(22) |
| 1-157 | 2 | $CF_3$ | $CO_2Me$ | H | H | c-Hex | Me | O | O | 1.4928(22) |
| 1-158 | 2 | $CF_3$ | $CO_2$-i-Bu | H | H | $CH_2OPh$ | Me | O | O | 1.5152(22) |
| 1-159 | 2 | $CF_3$ | $CO_2Me$ | H | H | $CH_2OPh$ | Me | O | O | 1.5052(22) |
| 1-160 | 2 | $CF_3$ | H. | H | H | $CH(CH_3)(CH_2)_3$ | | O | O | 81.1-820 |
| 1-161. | 2 | $CF_3$ | $CO_2$-i-Bu | H | H | $CH(CH_3)(CH_2)_3$ | | O | O | 1.4862(22) |
| 1-162 | 2 | $CF_3$ | $CO_2Me$ | H | H | $CH(CH_3)(CH_2)_3$ | | O | O | 1.5010(22) |
| 1-163 | 2 | $CF_3$ | $CH_2CO_2Et$ | H | H | Me | Me | O | O | 125.7-128.4 |
| 1-164 | 2 | $CF_3$ | H | H | H | $CH=C(CH_3)_2$ | Me | O | O | 109.3-110.0 |
| 1-165 | 2 | $CF_3$ | $CO_2$-i-Bu | H | H | $CH=C(CH_3)_2$ | Me | O | O | 1.4912(22) |
| 1-166 | 2 | $CF_3$ | $CO_2Me$ | H | H | $CH=C(CH_3)_2$ | Me | O | O | 1.4966(23) |
| 1-167 | 2 | $CF_3$ | H | H | H | $C(CH_2)_2CH_3$ | Me | O | O | 1.4896(22) |
| 1-168 | 2 | $CF_3$ | $CO_2$-i-Bu | H | H | $C(CH_2)_2CH_3$ | Me | O | O | 1.4810(22) |
| 1-169 | 2 | $CF_3$ | $CO_2Me$ | H | H | $C(CH_2)_2CH_3$ | Me | O | O | 1.4878(22) |
| 1-170 | 2 | $CF_3$ | H | H | H | c-Pr | c-Pr | O | O | 1.4940(23) |
| 1-171 | 2 | $CF_3$ | $CO_2$-i-Bu | H | H | c-Pr | c-Pr | O | O | 1.4970(23) |
| 1-172 | 2 | $CF_3$ | $CO_2Me$ | H | H | c-Pr | c-Pr | O | O | 95.4-99.0 |
| 1-173 | 2 | $CF_3$ | H | H | H | $CH_2OBn$ | Me | O | O | 98.3-99.5 |
| 1-174 | 2 | $CF_3$ | $CO_2$-i-Bu | H | H | $CH_2OBn$ | Me | O | O | 1.5106(23) |
| 1-175 | 2 | $CF_3$ | $CO_2Me$ | H | H | $CH_2OBn$ | Me | O | O | 1.5078(23) |

(continued)

Table 1 (X=$R^3$=$R^4$=H)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-176 | 2 | $CF_3$ | H | H | H | Q2 | Me | O | O | 143.7-147.8 |
| 1-177 | 2 | $CF_3$ | $CO_2$-i-Bu | H | H | Q2 | Me | O | O | 1.4798(23) |
| 1-178 | 2 | $CF_3$ | H | H | H | Et | Et | O | S | 97.8-98.5 |
| 1-179 | 2 | $CF_3$ | $CO_2$-i-Bu | H | H | Et | Et | 0 | S | 111.7-112.9 |
| 1-180 | 2 | $CF_3$ | H | H | H | Et | Me | O | S | 78-79 |
| 1-181 | 2 | $CF_3$ | $CO_2$+Bu | H | H | Et | Me | O | S | 982 |
| 1-182 | 2 | $CF_3$ | $CO_2Me$ | H | H | Et | Me | O | S | 138.8 |
| 1-183 | 2 | $CF_3$ | $CO_2Me$ | H | H | Et | Et | O | S | 144.8-144.9 |
| 1-184 | 2 | $CF_3$ | H | H | H | Me | Me | S | 0 | 115.0-117.5 |
| 1-185 | 2 | $CF_3$ | $CO_2$-i-Bu | H | H | Me | Me | S | O | 1.4978(26) |
| 1-186 | 2 | $CF_3$ | $CO_2Me$ | H | H | Me | Me | S | O | 1.5088(22) |
| 1-187 | 2 | $CF_3$ | H | O | | Me | Me | O | O | NMR |
| 1-188 | 2 | $CF_3$ | $CO_2Me$ | O | | Me | Me | O | O | 94.0-95.5 |
| 1-189 | 2 | $CF_3$ | $CO_2$-i-Bu | O | | Me | Me | O | O | 1.4867(15) |
| 1-190 | 2 | $CF_3$ | H | O | | Me | H | O | O | 1.4886(24) |
| 1-191 | 2 | $CF_3$ | $CO_2$-i-Bu | O | | Me | H | O | O | 1.4802(24) |
| 1-192 | 2 | $CF_3$ | H | O | | Et | H | O | O | 1.4970(22) |
| 1-193 | 2 | $CF_3$ | $CO_2$-i-Bu | O | | Et | H | O | O | 1.4868(21 ) |
| 1-194 | 2 | $CF_3$ | H | OH | H | Me | Me | O | 0 | 88-92 |
| 1-195 | 2 | $CF_3$ | H | OMe | H | Me | Me | O | O | NMR |
| 1-196 | 2 | $CF_3$ | $CO_2$-n-Bu | OMe | H | Me | Me | O | O | 1.4800(26) |
| 1-197 | 2 | $CF_3$ | $CO_2$-i-Bu | OMe | H | Me | Me | O | O | 1.4794(26) |
| 1-198 | 2 | $CF_3$ | H | OEt | H | Me | Me | O | O | 116-117 |
| 1-199 | 2 | $CF_3$ | H | O | | Me | H | S | O | 1.5108(26) |
| 1-200 | 2 | $CF_3$ | $CO_2$-i-Bu | O | | Me | H | S | O | 1.4858(26) |
| 1-201 | 2 | $CF_3$ | H | H | H | Ph | c-Pr | O | O | 1.5202(22) |
| 1-202 | 2 | $CF_3$ | $CO_2$+Bu | H | H | Ph | c-Pr | O | O | 1.5206(21) |
| 1-203 | 2 | $CF_3$ | $CO_2Me$ | H | H | Ph | c-Pr | O | O | 1.5156(21) |
| 1-204 | 2 | $CF_3$ | H | H | H | Ph | $CF_3$ | O | O | 101.3-101.6 |
| 1-205 | 2 | $CF_3$ | $CO_2$-i-Bu | H | H | Ph | $CF_3$ | O | O | 1.4962(21) |
| 1-206 | 2 | $CF_3$ | $CO_2Me$ | H | H | Ph | $CF_3$ | O | O | 1.4958(21) |

(continued)

Table 1 ($X=R^3=R^4=H$)

| Compound No. | Position of the substituent | $R^1$ | $R^2$ | $R^5$ | $R^6$ | $R^7$ | $R^8$ | A | W | Properties |
|---|---|---|---|---|---|---|---|---|---|---|
| 1-207 | 2 | $CF_3$ | H | H | - | | Me | O | O | 1202-121.2 |
| 1-208 | 2 | $CF_3$ | $CO_2$-i-Bu | H | - | | Me | O | O | 60.3-68.6 |
| 1-209 | 2 | $CF_3$ | H | H | - | | Et | O | O | 102.6-102.7 |
| 1-210 | 2 | $CF_3$ | $CO_2$-i-Bu | H | - | | Et | O | O | 70.9-73.0 |

[Table 2]

General formula (I)

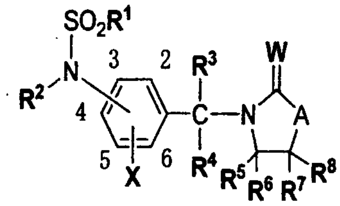

( I )

Table 2 ($R^1$=$CF_3$, $R^3$=$R^4$=H, W=A=O)

| Compound No. | Position of the substituent | X | $R^2$ | $R^5$ | $R^6$ | $R^7$ | $R^8$ | Properties |
|---|---|---|---|---|---|---|---|---|
| 1-211 | 2 | 5-Cl | H | O | | Me | Me | 90-91 |
| 1-212 | 2 | 3-$OCH_2$OMe | H | H | H | Me | Me | 98-101 |
| 1-213 | 2 | 3-$OCH_2$OMe | $CO_2$+Bu | H | H | Me | Me | 93.5-95.5 |
| 1-214 | 2 | 3-OH | H | H | H | Me | Me | 162-165 |
| 1-215 | 2 | 4,5-$OCH_2$O | H | H | H | Me | Me | 128.5 |
| 1-216 | 2 | 4,5-$OCH_2$O | $CO_2$Et | H | H | Me | Me | 178.4 |
| 1-217 | 2 | 4,5-$OCH_2$O | $CO_2$-i-Bu | H | H | Me | Me | 1.4865(24) |
| 1-218 | 2 | 6-F | H | O | | Me | Me | NMR |

[Table 3]

General formula (IV)

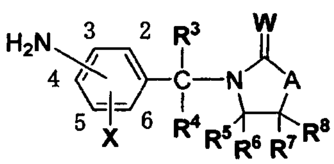

( IV )

Table 3 (X=$R^3$=$R^4$=H)

| Compound No. | Position of the substituent | $R^5$ | $R^6$ | $R^7$ | $R^8$ | A | W | Properties |
|---|---|---|---|---|---|---|---|---|
| 2-1 | 2 | Me | H | H | H | O | O | NMR |
| 2-2 | 2 | Et | H | H | H | O | O | 1.5610(23) |
| 2-3 | 2 | n-Pr | H | H | H | O | O | 1.5472(23) |
| 2-4 | 2 | i-Pr | H | H | H | O | O | 1.5478(20) |
| 2-5 | 2 | n-Bu | H | H | H | O | O | 1.5379(22) |
| 2-6 | 2 | $CO_2$Me | H | H | H | O | O | 1.5522(20) |
| 2-7 | 2 | Me | Me | H | H | O | O | 73-76 |
| 2-8 | 2 | H | H | Me | H | O | O | 1.5468(22) |

(continued)

Table 3 (X=$R^3$=$R^4$=H)

| Compound No. | Position of the substituent | $R^5$ | $R^6$ | $R^7$ | $R^8$ | A | W | Properties |
|---|---|---|---|---|---|---|---|---|
| 2-9 | 2 | H | H | $CF_3$ | H | O | O | 92.2-92.6 |
| 2-10 | 2 | H | H | Me | H | O | S | 1.6092(22) |
| 2-11 | 2 | H | H | Et | H | O | O | NMR |
| 2-12 | 3 | H | H | Et | H | O | O | 1.5431(23) |
| 2-13 | 4 | H | H | Et | H | O | O | 93.3 |
| 2-14 | 2 | H | H | n-Pr | H | O | O | 1.5394(21) |
| 2-15 | 2 | H | H | i-Pr | H | O | O | 1.5298(21) |
| 2-16 | 2 | H | H | t-Bu | H | O | O | 1.5320(23) |
| 2-17 | 2 | H | H | $CH_2OMe$ | H | O | O | 1.5392(23) |
| 2-18 | 2 | H | H | $CH_2OAc$ | H | O | O | NMR |
| 2-19 | 2 | H | H | $CH_2Cl$ | H | O | O | 1.5558(22) |
| 2-20 | 2 | H | H | Me | Me | O | O | NMR |
| 2-21 | 2 | H | H | $(CH_2)_5$ | | O | O | 1.5518(20) |
| 2-22 | 2 | H | $(CH_2)_4$ | | H | O | O | 105.3-107.6 |
| 2-23 | 2 | H | H | Me | Me | O | S | 77.7-78.6 |
| 2-24 | 2 | H | H | $CH_2OPh$ | Me | O | O | 122.4-124.0 |
| 2-25 | 2 | H | H | Et | H | O | O | 130.1-131.5 |
| 2-26 | 2 | H | H | Et | H | O | O | 130.3-131.8 |
| 2-27 | 2 | H | H | Et | Me | O | O | 722-74.7 |
| 2-28 | 2 | H | H | Et | Et | O | O | 1.5350(23) |
| 2-29 | 2 | H | H | t-Bu | Me | O | O | 1.5246(22) |
| 2-30 | 2 | H | H | c-Pr | Me | O | O | 1.5402(22) |
| 2-31 | 2 | Me | Me | Me | Me | O | O | 1.5376(23) |
| 2-32 | 2 | H | H | $(CH_2)_3$ | | O | O | 102.9-107.3 |
| 2-33 | 2 | H | H | $(CH_2)_2$ | | O | O | 1.5690(23) |
| 2-34 | 2 | H | Me | Me | Me | O | O | 81.6-83.5 |
| 2-35 | 2 | H | H | i-Pr | Me | O | O | 1.5256(23) |
| 2-36 | 2 | H | H | i-Bu | Me | O | O | NMR |
| 2-37 | 2 | H | H | $CH_2NHCO_2$-t-Bu | H | O | O | 141.9-142.1 |
| 2-38 | 2 | H | H | n-Pr | Me | O | O | 1.5344(27) |
| 2-39 | 2 | H | H | n-Bu | Me | O | O | 1.5288(27) |
| 2-40 | 2 | H | H | n-Pr | Et | O | O | 1.5216(27) |
| 2-41 | 2 | H | H | i-Pr | Et | O | O | 1.5238(27) |
| 2-42 | 2 | H | H | $CF_3$ | Me | O | O | 1.5038(26) |
| 2-43 | 2 | H | H | $CH_2OMe$ | Me | O | O | 1.5332(24) |
| 2-44 | 2 | H | H | Ph | Me | O | O | 1.5634(23) |
| 2-45 | 2 | H | H | c-Hex | Me | O | O | 1.5436(21) |
| 2-46 | 2 | H | H | $CH_2OPh$ | Me | O | O | 114.6 |
| 2-47 | 2 | H | H | $CH(CH_3)(CH_2)_3$ | | O | O | 1.5542(21) |
| 2-48 | 2 | H | H | $CH=C(CH_3)_2$ | Me | O | O | 1.5364(21) |
| 2-49 | 2 | H | H | $C(CH_2)_2CH_3$ | Me | O | O | 1.5372(21) |
| 2-50 | 2 | H | H | c-Pr | c-Pr | O | O | 1.5338(22) |
| 2-51 | 2 | H | H | $CH_2OBn$ | Me | O | O | 1.5572(22) |
| 2-52 | 2 | H | H | Et | Et | O | S | 1.5726(23) |
| 2-53 | 2 | H | H | Et | Me | O | S | 69-70 |
| 2-54 | 2 | H | H | Me | Me | S | O | 1.5788(25) |

(continued)

| Table 3 ($X=R^3=R^4=H$) | | | | | | | | |
|---|---|---|---|---|---|---|---|---|
| Compound No. | Position of the substituent | $R^5$ | $R^6$ | $R^7$ | $R^8$ | A | W | Properties |
| 2-55 | 2 | O | | Me | Me | S | O | 105.9-107.2 |
| 2-56 | 2 | O | | Me | Me | O | O | 122.5-123.5 |
| 2-57 | 2 | O | | Me | H | O | O | 1.5458(23) |
| 2-58 | 2 | O | | Et | H | O | O | 1.5488(23) |
| 2-59 | 2 | OMe | H | Me | Me | O | O | NMR |
| 2-60 | 2 | H | H | Ph | c-Pr | O | O | 1.5758(23) |
| 2-61 | 2 | H | H | Ph | $CF_3$ | O | O | 1.5430(22) |
| 2-62 | 2 | H | - | | Me | O | O | 99.5-101.9 |
| 2-63 | 2 | H | - | | Et | O | O | 1.5548(23) |

[Table 4]

General formula (IV)

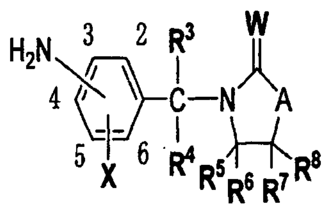

( IV )

| Table 4 ($R^3=R^4=H$, A=W=O) | | | | | | | |
|---|---|---|---|---|---|---|---|
| Compound No. | Position of the substituent | X | $R^5$ | $R^6$ | $R^7$ | $R^8$ | Properties |
| 2-64 | 2 | 5-Cl | O | | Me | Me | 120.5-121.5 |
| 2-65 | 2 | 3-$OCH_2OMe$ | H | H | Me | Me | NMR |
| 2-66 | 2 | 4,5-$OCH_2O$ | H | H | Me | Me | 1.5342(26) |
| 2-67 | 2 | 6-F | O | | Me | Me | 98-99 |

[Table 5]

| Table 5 | |
|---|---|
| Compound No. | $^1$H-NMR($CDCl_3$/TMS, δ ppm) |
| 1-106 | 7.59(dd 1H), 7.40(dd, 1H), 7.28-7.22(m, 2H), 4.90(br. 1H), 4.66(m, 1H), 4.45(d, 1H), 4.36(d, 1H), 3.68(dd, 1H), 3.46-3.33(m, 3H), 1.40(s, 9H) |
| 1-187 | 9.30(brs, 1H), 7.56(d, 1H), 7.53(d, 1H), 7.43(dd, 1H), 7.32(dd, 1H), 4.74(s, 2H), 1.56(s, 6H) |
| 1-195 | 10.0(brs, 1H), 7.58(d, 1H), 7.25-7.41(m, 3H), 4.51(s, 1H), 4.42(AB, 2H), 3.51(s,3H), 1.42(s, 3H), 1.36(s, 3H) |
| 1-218 | 9.42(brs, 1H), 7.37-7.46(m, 2H), 7.11(dd, 1H), 4.80(s, 2H), 1.58(s, 6H) |
| 2-1 | 7.13(1H, dd), 7.03(1H, d), 6.66(2H, m), 4.70(1H, d), 4.37(1H, dd), 4.32(2H, brs), 4.06(1H, d), 3.83 (1H, dd), 3.70(1H, dd), 1.29(3H, d) |

(continued)

| Table 5 | |
|---|---|
| Compound No. | $^1$H-NMR($CDCl_3$/TMS, δ ppm) |
| 2-11 | 7.13(1H, dd), 7.00(1H, d), 6.66(2H, m), 4.46-4.32(3H, m), 4.28(2H, brs), 3.48(1H, dd). 3.03(1H. dd), 1.17-1.59(2H. m), 0.95(3H, t) |
| 2-18 | 7.14(1H, dd), 7.01(1H, d), 6.68(2H, m), 4.70(1H, m), 4.39(1H, d). 4.32(1H, d), 4.18(2H, m), 3.54 (1H, dd), 320 (1H, dd), 2.01(3H, s) |
| 2-20 | 7.13(1H, dd), 6.99(1H, d), 6.67(2H, m), 4.35(2H, s), 4.27(2H, brs), 3.14(2H, s), 1.40(6H, s) |
| 2-36 | 7.13(dt, 1H), 6.98(dd. 1H). 6.83-6.64(m, 2H), 4,36(d, 1H), 4.28(d, 1H), 4.27(brs, 2H), 3.17(d. 1H), 3.07(d, 1H), 1.75(m, 1H), 1.61-1.49(m, 2H), 1.37(s, 3H), 0.94(d, 3H), 0.89(d, 3H) |
| 2-59 | 7.14(1H, dd), 7.05(1H, d), 6.69(1H, dd), 6.68(1H, d), 4.75(1H, d), 4.13(1H, s), 4.12(2H, brs), 4.07 (1H,d), 3.38(3H, s), 1.40(3H, s), 1.27(3H, s) |
| 2-65 | 7.01(dd, 1H), 6.70(dd, 1H), 6.59(t, 1H), 5.20(s, 2H), 4.50(brs, 2H), 4.37(s, 2H), 3.50(s, 3H), 3.15 (s, 2H), 1.40(s, 6H) |

The herbicides comprising, as an active ingredient, the haloalkyl sulfonanilide derivative represented by the formula (I) or salt thereof of the present invention are useful for controlling annual, biennial and perennial weeds which grow in paddy fields, upland fields, orchards, swamps, etc., such as barnyard grass (*Echinochloa crus-qalli* Beauv., an annual gramineous grass which is an injurious weed of paddy fields), false pimpernel (Lindernia pyxidaria, an annual scrophulariaceous weed which is an injurious weed of paddy fields), monochoria (Monochoria vaginalis, an annual pontederiaceous weed which is an injurious weed of paddy fields), Monochoria korsakowii (an annual pontederiaceous weed which is an injurious weed of paddy fields), ammannia (Ammannia multiflora Roxb., an annual lythraceous weed which is an injurious weed of paddy fields), smallflower umbrella sedge (Cyperus difformis L., an annual cyperaceous weed which is an injurious weed of paddy fields), needle spikerush (Eleocharis acicularis Roem. et Schult, a perennial cyperaceous weed which is an injurious weed of paddy fields and which grows also in swamps and waterways), arrowhead (Sagittaria trifolia L., an injurious perennial weed of Alismataceae family which grows in paddy fields, swamps and ditches), Sagittaria pygmaea Miq., (an injurious perennial weed of Alismataceae family which grows in paddy fields, swamps and ditches), bulrush (Scirpus juncoides Roxb. subsp. juncoides Roxb., a perennial cyperaceous weed which grows in paddy fields, swamps and ditches), Eleocharis kuroguwai (a perennial cyperaceous weed which grows in paddy fields, swamps and ditches), foxtail grass (Alopecurus aequalis var. amurensis Ohwi, biennial gramineous grass which grows in upland fields and low swamps), wild oat (Avena fatua L., a biennial gramineous grass which grows in plains, waste lands and upland fields), mugwort (Artemisia princeps Pamp., a perennial composite weed which grows in cultivated and uncultivated fields and mountains), large crabgrass (Digitaria adscendens Henr., an annual gramineous grass which is an injurious weed of upland fields and orchards), Gishigishi or Japanese dock (Rumex japonicus Houtt., a perennial polygonaceous weed which grows in upland fields and roadsides), flatsedge (Cyperus iria L., an annual cyperaceous weed which is an injurios weed of upland fields), slender amaranth (Amaranthus viridis L., an annual weed of Amaranthaceae family which grows in vacant lands, roadsides and upland fields), cocklebur (Xanthium strumarium L., an injurious annual composite weed which grows in upland fields), velvetleaf (Abutilon theophrasti L., an injurious annual weed of Malvaceae family which grows in upland fields), jimsonweed (Datura stramonium L., an annual solanaceous weed which is an injurious weed of upland fields), birdseye speedwell (Veronica persica Poir., an injurious biennial weed of Scrophulariaceae family which grows in upland fields) and cleavers (Galium spurium L., an injurious biennial weed of Rubiaceae family which grows in upland fields and orchards), etc. Particularly, herbicides comprising the haloalkyl sulfonanilide derivative or salt thereof of the present invention are useful for controlling weeds in paddy fields. The herbicides according to the present invention have a broad selectivity between paddy rice and injurious weeds of paddy fields, thus, the herbicides of the present invention exhibit an excellent effect as herbicides for controlling weeds in paddy fields.

Since the herbicides comprising, as an active ingredient, the haloalkyl sulfonanilide derivative represented by the formula (I) or salt thereof exhibit an excellent controlling effect on weeds pre- or post-emergence, the characteristic physiological activities of the herbicides can be effectively manifested by treating fields with the herbicides before planting useful plants therein, or after planting useful plants therein (including the case in which useful plants are already planted as in orchards) but during the period from the initial stage of emergence of weeds to their growth stage.
However, the application of the herbicides of the present invention is not restricted only to the modes mentioned above. The herbicides of the present invention can be applied to control not only weeds which grow in paddy fields but also weeds which grow in other places such as vestiges of mowing, temporarily non-cultivated paddy fields and upland fields,

ridges between fields, agricultural pathways, waterways, lands constructed for pasture, graveyards, parks, roads, playgrounds, unoccupied areas around buildings, developed lands, railways, forests and the like.
The treatment of target fields with the herbicides is most effective in economy when the treatment is made by the initial stage of emergence of weeds. However, the treatment is not restricted thereto and can be carried out even during the growth stage of weeds.
For applying the haloalkyl sulfonanilide derivative represented by the formula (I) or salt thereof as herbicides, they are generally formulated into a form convenient to use according to the procedure conventionally employed for preparing agricultural chemicals.
That is, the haloalkyl sulfonanilide derivative represented by the formula (I) or salt thereof is mixed with a suitable inert carrier and, as necessary, further with an adjuvant, in an appropriate ratio, and the mixture is made into a desired form of preparation, such as suspension, emulsion, emulsifiable concentrate, solution, wettable powder, water dispersible granule, granules, dust, tablets, jumbo, packs and the like, through dissolution, dispersion, suspension, mixing, impregnation, adsorption or adhesion.

The inert carriers usable in the present invention may be solid or liquid. Materials usable as the solid carriers include, for example, vegetable powders (e.g. soybean flour, cereal flour, wood flour, bark flour, saw dust, powdered tobacco stalks, powdered walnut shells, bran, powdered cellulose and extraction residues of vegetables), synthetic polymers such as powdered synthetic resins, clays (e.g. kaolin, bentonite and acid clay), talcs (e.g. talc and pyrophyllite), silica powders or flakes [e.g. diatomaceous earth, silica sand, mica and white carbon (i.e. highly dispersed silicic acid, also called finely divided hydrated silica or hydrated silicic acid)], activated carbon, natural mineral materials (e.g. powdered sulfur, powdered pumice, attapulgite and zeolite), calcined diatomaceous earth, ground brick, fly ash, sand, plastic carriers (e.g. polyethylene, polypropylene and polyvinylidene chloride), inorganic mineral powders (e.g. calcium carbonate powder, calcium phosphate powder, etc.), chemical fertilizers (e.g. ammonium sulfate, ammonium phosphate, ammonium nitrate, urea, and ammonium chloride) and compost. These materials can be used alone or in combination of two or more.
Materials usable as the liquid carriers are selected not only from those which have solvency by themselves but also from those which have no solvency but capable of dispersing the active ingredient compound with the aid of adjuvants. Typical examples of the liquid carriers, which can be used alone or in combination of two or more, are water, alcohols (e.g. methanol, ethanol, isopropanol, butanol and ethylene glycol), ketones (e.g. acetone, methyl ethyl ketone, methyl isobutyl ketone, diisobutyl ketone and cyclohexanone), ethers (e.g. ethyl ether, dioxane, Cellosolve, dipropyl ether and tetrahydrofuran), aliphatic hydrocarbons (e.g. kerosene and mineral oils), aromatic hydrocarbons (e.g. benzene, toluene, xylene, solvent naphtha and alkylnaphthalenes), halogenated hydrocarbons (e.g. dichloroethane, chloroform and carbon tetrachloride), esters (e.g. ethyl acetate, diisopropyl phthalate, dibutyl phthalate and dioctyl phthalate), amides (e.g. dimethylformamide, diethylformamide and dimethylacetamide), nitriles (e.g. acetonitrile), and dimethyl sulfoxide.

As the adjuvants, there can be mentioned the following typical adjuvants. They are used according to respective purpose. They may be used alone or in combination of two or more, or may not be used at all.
For the purpose of emulsifying, dispersing, solubilizing and/or wetting the active ingredient compounds, there are used surface active agents, for example, polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.
For the purpose of imparting stable dispersion, tackiness and/or bonding property to the active ingredient compounds, there may be used adjuvants such as casein, gelatin, starch, methyl cellulose, carboxymethyl cellulose, gum arabic, polyvinyl alcohol, turpentine, bran oil, bentonite and ligninsulfonates.
For the purpose of improving the flow properties of solid herbicidal compositions, there may be used adjuvants such as waxes, stearates and alkyl phosphates.
Adjuvants such as naphthalenesulfonic acid condensation products and polyphosphates may be used as peptizers in dispersible herbicidal compositions.
Adjuvants such as silicone oils may be used as defoaming agent.

Although the content of the active ingredient compound may be varied as occasion demands and there is no specific limitation for the content, it may be 0.01 to 90% by weight generally. For example, for the preparation of a powdered or granulated product, the content is preferably 0.01 to 50% by weight, and more preferably 0.1 to 10% by weight. For the preparation of an emulsifiable concentrate, wettable powder or granulated wettable powder, the content is also suitably 0.01 to 90% by weight as well, and more suitably 0.01 to 60% by weight.
For controlling various weeds or inhibiting their growth, the herbicides comprising, as an active ingredient, the haloalkyl sulfonanilide derivative represented by the formula (I) or salt thereof are applied as such or after appropriately diluted with or suspended in water or other media, in an amount effective for controlling weeds or inhibiting their growth, to the foliage and stalks of the weeds or to soil in the area where the emergence or growth of the weeds is undesirable.
The usable amount of herbicides comprising, as an active ingredient, the haloalkyl sulfonanilide derivative represented

by the formula (I) or salt thereof varies depending on various factors, for example, the purpose of application, the kinds of target weeds, the growth states of crops, the emergence tendency of weeds, weather, environmental conditions, the form of the herbicides used, the mode of application, the type or state of application site and the time of application. However, the amount is selected appropriately according to the purpose from the range of 0.1 g to 10 kg in terms of the amount of active ingredient compound per hectare.
The herbicides containing, as an active ingredient, the haloalkyl sulfonanilide derivative represented by the formula (I) or salt thereof can be applied jointly with other herbicides for the purpose of expanding both the spectrum of controllable weeds and the period of time when effective application is possible or for the purpose of reducing the dosage.

Next, the present invention will be described particularly using examples, but the present invention is not limited to these.

Example 1. Production of 4-ethyl-3-[2-(trifluoromethanesulfonylamino)benzyl]-2-oxazolidinone (Compound No. 1-5)

1-1) Production of 4-ethyl-3-(2-nitrobenzyl)-2-oxazolidinone

2-(2-nitrobenzylamino)-1-butanol (3.2 g, 14 mmol) was dissolved in chloroform (100 ml) and the reaction solution was cooled to 0˚C. Next, triethylamine (7.1 g, 71 mmol) and triphosgen (2.1 g, 7.1 mmol) were added. The reaction solution was stirred at 0˚C for 1.5 hour and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 4-ethyl-3-(2-nitrobenzyl)-2-oxazolidinone (2.6 g).
Yield: 72%
Properties: $n_D$ 1.5514 (23˚C)

1-2) Production of 4-ethyl-3-(2-aminobenzyl)-2-oxazolidinone (Compound No. 2-2)

4-ethyl-3-(2-nitrobenzyl)-2-oxazolidinone (2.6 g, 10 mmol), iron powder (2.8 g, 51 mmol), ammonium chloride (0.3 g, 5.1 mmol) were dissolved in ethanol (30 ml) and water (15 ml), and the solution was refluxed for 1 hour. After cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 4-ethyl-3-(2-aminobenzyl)-2-oxazolidinone (2.0 g).
Yield: 88%
Properties: $n_D$ 1.5610 (23˚C)

1-3) Production of 4-ethyl-3-[2-(trifluoromethanesulfonylamino)benzyl]-2-oxazolidinone (Compound No. 1-5)

4-ethyl-3-(2-aminobenzyl)-2-oxazolidinone (2.0 g, 9.0 mmol) and triethylamine (1.4 g, 13 mmol) were dissolved in chloroform (25 ml) and then the reaction solution was cooled to -30˚C. To the reaction solution, trifluoromethanesulfonic anhydride (3.0 g, 11 mmol) was added dropwise, and the solution was stirred at -30˚C for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 4-ethyl-3-[2-(trifluoromethanesulfonylamino)benzyl]-2-oxazolidinone (2.2 g).
Yield: 69%
Properties: melting point 81.4-83.6˚C

Example 2. Production of 4-ethyl-3-{2-[N-(isobutoxycarbonyl)-N-(trifluoromethanesulfonyl)amino]benzyl}-2-oxazolidinone (Compound No. 1-6)

4-ethyl-3-[2-(trifluoromethanesulfonylamino)benzyl]-2-oxazolidinone (1.0 g, 2.8 mmol) and sodium bicarbonate (0.48 g, 5.7 mmol) were suspended in acetonitrile (10 ml) and isobutyl chloroformate (0.78 g, 5.7 mmol) was added and refluxed for 2 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:2) to obtain 4-ethyl-3-{2-[N-(isobutoxycarbonyl)-N-(trifluoromethanesulfonyl)amino]benzyl}-2-oxazolidinone (1.2 g).
Yield: 91%
Properties: melting point 69.3-71.7˚C

Example 3. Production of 4,4-dimethyl-3-[2-(trifluoromethanesulfonylamino)benzyl]-2-oxazolidinone (Compound No. 1-15)

3-1) Production of 4,4-dimethyl-3-(2-nitrobenzyl)-2-oxazolidinone

2-(2-nitrobenzylamino)-2-methyl-1-propanol (2.6 g, 12 mmol) was dissolved in chloroform (100 ml) and the reaction solution was cooled to 0˚C, and then triethylamine (5.9 g, 59 mmol) and triphosgene (1.7 g, 5.9 mmol) were added. The reaction solution was stirred at that temperature for 1.5 hour and then extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 4,4-dimethyl-3-(2-nitrobenzyl)-2-oxazolidinone (1.3 g).
Yield: 43%
Properties: melting point 103-106˚C

3-2) Production of 4,4-dimethyl-3-(2-aminobenzyl)-2-oxazolidinone (Compound No. 2-7)

4,4-dimethyl-3-(2-nitrobenzyl)-2-oxazolidinone (0.75 g, 3.0 mmol), iron powder (0.84 g, 15 mmol), ammonium chloride (0.08 g, 1.5 mmol) were dissolved in ethanol (20 ml) and water (10 ml), and the solution was heated and refluxed for 1 hour. After cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 4,4-dimethyl-3-(2-aminobenzyl)-2-oxazolidinone (0.66 g).
Yield: 100%
Properties: melting point 73-76˚C

3-3) Production of 4,4-dimethyl-3-[2-(trifluoromethanesulfonylamino)benzyl]-2-oxazolidinone (Compound No. 1-15)

4,4-dimethyl-3-(2-aminobenzyl)-2-oxazolidinone (0.66 g, 3.0 mmol) and triethylamine (0.45 g, 4.5 mmol) were dissolved in chloroform (25 ml) and then the reaction solution was cooled to -30˚C. To the reaction solution, trifluoromethanesulfonic anhydride (1.0 g, 3.6 mmol) was added dropwise, and the solution was stirred at that temperature for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 4,4-dimethyl-3-[2-(trifluoromethanesulfonylamino)benzyl]-2-oxazolidinone (0.87 g).
Yield: 82%
Properties: melting point 134.9˚C

Example 4. Production of 4,4-dimethyl-3-{2-[N-(isobutoxycarbonyl)-N-(trifluoromethanesulfonyl)amino]benzyl}-2-oxazolidinone (Compound No. 1-16)

4,4-dimethyl-3-[2-(trifluoromethanesulfonylamino)benzyl]-2-oxazolidinone (0.35 g, 0.99 mmol) and sodium bicarbonate (0.17 g, 2.0 mmol) were suspended in acetonitrile (20 ml) and isobutyl chloroformate (0.27 g, 2.0 mmol) was added and refluxed for 2 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography(ethylacetate:hexane=1:2)toobtain4,4-dimethyl-3-{2-[N-(isobutoxycarbonyl)-N-(trifluoromethanesulfonyl)amino]benzyl}-2-oxazolidinone (0.43 g).
Yield: 90%
Properties: melting point 65.0-65.8˚C

Example 5. Production of 5-methyl-3-(2-trifluoromethanesulfonylaminobenzyl)-2-oxazolidinethione (Compound No. 1-21)

5-1) Production of 5-methyl-3-(2-nitrobenzyl)-2-oxazolidinethione.

1-(2-nitrobenzylamino)-2-propanol (1.1 g, 5.2 mmol) was dissolved in chloroform (30 ml) and the reaction solution was cooled to 0˚C, and then triethylamine (1.6 g, 16 mmol) and thiophosgene (0.78 g, 6.8 mmol) were added. The reaction solution was stirred at 0˚C for 1.5 hour and extracted with ethyl acetate after adding water. The ethyl acetate layer was washed with saturated sodium chloride solution, and then dried with anhydrous magnesium sulfate and the

solvent was removed by distillation. The residue was purified by silica gel column chromatography (ethyl acetate:hexane = 1:1) to obtain 5-methyl-3-(2-nitrobenzyl)-2-oxazolidinethione (0.56 g).
Yield: 42%
Properties: melting point 137-139˚C

5-2) Production of 5-methyl-3-(2-aminobenzyl)-2-oxazolidinethione (Compound No. 2-10)

5-methyl-3-(2-nitrobenzyl)-2-oxazolidinethione (0.40 g, 1.6 mmol), iron powder (0.44 g, 7.9 mmol), ammonium chloride (0.04 g, 0.79 mmol) were dissolved in ethanol (15 ml) and water (7.5 ml), and the solution was heated and refluxed for 1 hour. After cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 5-methyl-3-(2-aminobenzyl)-2-oxazolidinethione (0.36 g).
Yield: 100%
Properties: $n_D$ 1.6092 (22˚C)

5-3) Production of 5-methyl-3-[2-(trifluoromethanesulfonylamino)benzyl]-2-oxazolidinethione (Compound No. 1-21)

5-methyl-3-(2-aminobenzyl)-2-oxazolidinethione (0.36 g, 1.6 mmol) and triethylamine (0.24 g, 2.4 mmol) were dissolved in chloroform (20 ml), and the reaction solution was cooled to -30˚C. To the reaction solution, trifluoromethanesulfonic anhydride (0.54 g, 1.9 mmol) was added dropwise, and the solution was stirred at -30˚C for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 5-methyl-3-[2-(trifluoromethanesulfonylamino)benzyl]-2-oxazolidinethione (0.49 g).
Yield: 87%
Properties: melting point 74-76˚C

Example 6. Production of 5-methyl-3-{2-[N-(isobutoxycarbonyl)-N-(trifluoromethanesulfonyl)-amino]benzyl}-2-oxazolidinethione (Compound No. 1-22)

5-methyl-3-[2-(trifluoromethanesulfonyl-amino)benzyl]-2-oxazolidinethione (0.33 g, 0.93 mmol) and sodium bicarbonate (0.16 g, 1.9 mmol) were suspended in acetonitrile (20 ml) and isobutyl chloroformate (0.25 g, 1.9 mmol) was added and refluxed for 2 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:2) to obtain 5-methyl-3-{2-[N-(isobutoxycarbonyl)-N-(trifluoromethanesulfonyl)amino]benzyl}-2-oxazolidinethione (0.37 g).
Yield: 88%
Properties: $n_D$ 1.5168 (22˚C)

Example 7. Production of 5-ethyl-3-[2-(trifluoromethanesulfonylamino)benzyl]-2-oxazolidinethione (Compound No. 1-23)

7-1) Production of 5-ethyl-3-(2-nitrobenzyl)-2-oxazolidinone

1-(2-nitrobenzylamino)-2-butanol (2.6 g, 12 mmol) was dissolved in chloroform (100 ml) and the reaction solution was cooled to 0˚C, and then pyridine (4.6 g, 59 mmol) and triphosgene (1.7 g, 5.9 mmol) were added. The reaction solution was stirred at that temperature for 1.5 hour and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 5-ethyl-3-(2-nitrobenzyl)-2-oxazolidinone (1.5 g).
Yield: 51%
Properties: no 1.5285 (24˚C)

7-2) Production of 5-ethyl-3-(2-aminobenzyl)-2-oxazolidinone (Compound No. 2-11)

5-ethyl-3-(2-nitrobenzyl)-2-oxazolidinone (1.5 g, 6.0 mmol), iron powder (1.7 g, 30 mmol), ammonium chloride (0.16 g, 3.0 mmol) were dissolved in ethanol (20 ml) and water (10 ml), and the solution was refluxed for 1 hour. After

cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 5-ethyl-3-(2-aminobenzyl)-2-oxazolidinone (1.3 g).
Yield: 100%
Property: 1H-NMR, [400 MHz, $CDCl_3$, δ (ppm)] 7.13 (dd, 1H), 7.00 (d, 1H), 6.66 (m, 2H), 4.46-4.32 (m, 3H), 4.28 (brs, 2H), 3.48 (dd, 1H), 3.03 (dd, 1H), 1.77-1.59 (m, 2H), 0.95 (t, 3H)

7-3) Production of 5-ethyl-3-[2-(trifluoromethanesulfonylamino)benzyl]-2-oxazolidinone (Compound No. 1-23)

5-ethyl-3-(2-aminobenzyl)-2-oxazolidinone (2.0 g, 6.0 mmol) and triethylamine (0.9 g, 8.9 mmol) were dissolved in chloroform (25 ml), and the reaction solution was cooled to -40˚C. To the reaction solution, trifluoromethanesulfonic anhydride (2.0 g, 7.1 mmol) was added dropwise, and the solution was stirred at -40˚C for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 5-ethyl-3-[2-(trifluoromethanesulfonylamino)benzyl]-2-oxazolidinone (1.5 g).
Yield: 71%
Properties: $n_D$ 1.4885 (23˚C)

Example 8. Production of 5-ethyl-3-{2-[N-(isobutoxycarbonyl)-N-(trifluoromethanesulfonyl)-amino]benzyl}-2-oxazolidinone (Compound No. 1-24)

5-ethyl-3-[2-(trifluoromethanesulfonyl-amino)benzyl]-2-oxazolidinone (0.35 g, 1.0 mmol) and sodium bicarbonate (0.13 g, 1.5 mmol) were suspended in acetonitrile (20 ml) and isobutyl chloroformate (0.20 g, 1.5 mmol) was added and refluxed for 2 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:2) to obtain 5-ethyl-3-{2-[N-(isobutoxycarbonyl)-N-(trifluoromethanesulfonyl)amino] benzyl}-2-oxazolidinone (0.23 g).
Yield: 51%
Properties: melting point 85-87˚C

Example 9. Production of 5-methyl-3-[2-(trifluoromethanesulfonylamino)benzyl]-4-oxazolin-2-one (Compound No. 1-207)

9-1) Production of 5-methyl-3-(2-nitrobenzyl)oxazolidine-2,4-dione

Sodium salt of 5-methyloxazolidine-2,4-dione (2.3 g, 17 mmol) was dissolved in dimethylformamide (50 ml), and 2-nitrobenzyl bromide (3.0 g, 14 mmol) was added and the reaction solution was heated at 60˚C for 30 min while stirring. Ice and saturated ammonium chloride solution was added to the reaction solution, which was extracted with ethyl acetate. After being washed with saturated sodium chloride solution, the ethyl acetate layer was dried with anhydrous magnesium sulfate and the solvent was removed by distillation. The residue was crystallized and washed with hexane-ether to obtain 5-methyl-3-(2-nitrobenzyl)oxazolidine-2,4-dione (3.3 g).
Yield: 95%
Properties: melting point 113.7-113.9˚C

9-2) Production of 4-hydroxy-5-methyl-3-(2-nitrobenzyl)oxazolidin-2-one

5-methyl-3-(2-nitrobenzyl)oxazolidine-2,4-dione (3.0 g, 20 mmol) was dissolved in tetrahydrofuran (50 ml), and lithium borohydride (0.40 g, 18 mmol) was added to the reaction solution cooled on ice. The reaction solution was stirred at room temperature for 8 hours. Ice and saturated ammonium chloride solution was added to the reaction solution, which was extracted with ethyl acetate. After being washed with saturated sodium chloride solution, the ethyl acetate layer was dried with anhydrous magnesium sulfate and the solvent was removed by distillation. The residue was crystallized and washed with hexane-ether to obtain 4-hydroxy-5-methyl-3-(2-nitrobenzyl)oxazolidin-2-one (2.6 g) as a mixture of diastereomer.
Yield: 86%
Property (Main product): $^1$H-NMR [400 MHz, $CDCl_3$,
δ (ppm)] 7.99 (d, 1H), 7.65-7.63 (m, 2H), 7.49 (t, 3H), 4.86 (dd, 1H), 4.81 (d, 1H), 4.80 (d, 1H), 4.44 (dq, 1H), 4.02 (d,

1H), 1.38 (d, 3H)

9-3) Production of 5-methyl-3-(2-nitrobenzyl)-4-oxazolin-2-one

4-hydroxy-5-methyl-3-(2-nitrobenzyl)oxazolidin-2-one (2.6 g, 10 mmol) was dissolved in tetrahydrofuran (50 ml), and thionyl chloride (1.2 g, 10 mmol) was added and the solution was refluxed for 1 hour. After cooled to room temperature, the reaction solution was mixed with ice and saturated sodium bicarbonate solution and extracted with ethyl acetate. The ethyl acetate layer was washed with saturated sodium chloride solution, and then dried with anhydrous magnesium sulfate and the solvent was removed by distillation. The residue was crystallized and washed with hexane-ether to obtain 5-methyl-3-(2-nitrobenzyl)-4-oxazolin-2-one (2.0 g).
Yield: 82%
Properties: melting point 82.9-83.9˚C

9-4) Production of 3-(2-aminobenzyl)-5-methyl-4-oxazolin-2-one (Compound No. 2-62)

5-methyl-3-(2-nitrobenzyl)-4-oxazolin-2-one (1.95 g, 8.33 mmol), iron powder (2.3 g, 42 mmol), ammonium chloride (0.22 g, 4.2 mmol) were dissolved in ethanol (25 ml) and water (13 ml), and the solution was refluxed for 1 hour. After cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 3-(2-aminobenzyl)-5-methyl-4-oxazolin-2-one (1.6 g).
Yield: 94%
Properties: melting point 99.5-101.9˚C

9-5) Production of 5-methyl-3-[2-(trifluoromethanesulfonylamino)benzyl]-4-oxazolin-2-one (Compound No. 1-207)

3-(2-aminobenzyl)-5-methyl-4-oxazolin-2-one (1.6 g, 7.8 mmol) and triethylamine (1.0 g, 10 mmol) were dissolved in chloroform (30 ml) and then the solution was cooled to -40˚C. To the reaction solution, trifluoromethanesulfonic anhydride (2.7 g, 9.4 mmol) was added dropwise, and the solution was stirred at -40˚C for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:1) to obtain 5-methyl-3-[2-(trifluoromethanesulfonylamino)benzyl]-4-oxazolin-2-one (2.1 g).
Yield: 79%
Properties: melting point 120.2-121.2˚C

Example 10. Production of 3-{2-[N-(isobutoxycarbonyl)-N-(trifluoromethanesulfonyl)amino]benzyl}-5-methyl-4-oxazolin-2-one (Compound No. 1-208)

5-methyl-3-[2-(trifluoromethanesulfonylamino)benzyl]-4-oxazolin-2-one (0.30 g, 0.89 mmol) and sodium bicarbonate (0.15 g, 1.8 mmol) were suspended in acetonitrile (20 ml) and isobutyl chloroformate (0.24 g, 1.8 mmol) was added and refluxed for 2 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column chromatography (ethyl acetate:hexane = 1:2) to obtain 3-{2-[N-(isobutoxycarbonyl)-N-(trifluoromethanesulfonyl)amino]benzyl}-5-methyl-4-oxazolin-2-one (0.23 g).
Yield: 59%
Properties: melting point 60.3-68.6˚C

Example 11. Production of 5,5-dimethyl-3-[2-(trifluoromethanesulfonylamino)benzyl]thiazolidin-2-one (Compound No. 1-184)

11-1) Production of 5,5-dimethyl-3-(2-nitrobenzyl)thiazolidine-2,4-dione

5,5-dimethyl-thiazolidine-2,4-dione (1.5 g, 10 mmol) was dissolved in dimethylformamide (30 ml) and 2-nitrobenzyl bromide (2.2 g, 10 mmol) was added and the reaction solution was heated at 60˚C for 30 min while stirring. Ice and saturated ammonium chloride solution was added to the reaction solution, which was extracted with ethyl acetate. After being washed with saturated sodium chloride solution, the ethyl acetate layer was dried with anhydrous magnesium sulfate and the solvent was removed by distillation. The residue was crystallized and washed with hexane-ether to obtain

5,5-dimethyl-3-(2-nitrobenzyl)thiazolidine-2,4-dione (2.8 g).
Yield: 99%
Properties: melting point 72.3-73.2°C

11-2) Production of 4-hydroxy-5,5-dimethyl-3-(2-nitrobenzyl)thiazolidin-2-one

5,5-dimethyl-3-(2-nitrobenzyl)thiazolidine-2,4-dione (2.0 g, 7.1 mmol) was dissolved in tetrahydrofuran (35 ml), and lithium borohydride (0.26 g, 11 mmol) was added to the reaction solution cooled on ice. The reaction solution was stirred at room temperature for 3.5 hours. Ice and saturated ammonium chloride solution was added to the reaction solution, which was extracted with ethyl acetate. After being washed with saturated sodium chloride solution, the ethyl acetate layer was dried with anhydrous magnesium sulfate and the solvent was removed by distillation. The residue was crystallized and the crude crystal was washed with hexane-ether to obtain 4-hydroxy-5,5-dimethyl-3-(2-nitrobenzyl) thiazolidin-2-one (2.0 g).
Yield: 99%
Properties: melting point 139.2-142.6°C

11-3) Production of 5,5-dimethyl-3-(2-nitrobenzyl)thiazolidin-2-one

4-hydroxy-5,5-dimethyl-3-(2-nitrobenzyl)thiazolidin-2-one (1.8 g, 6.5 mmol) was dissolved in chloroform (30 ml), and the solution was mixed with triethyl silane (1.1 g, 9.7 mmol) and trifluoroacetic acid (7.4 g, 65 mmol) on ice. The reaction solution was stirred at room temperature for 10 hours. After the solvent of the reaction solution was removed by distillation under reduced pressure, the residue was mixed with saturated sodium bicarbonate solution and extracted with ethyl acetate. After being washed with saturated sodium chloride solution, the ethyl acetate layer was dried with anhydrous magnesium sulfate and the solvent was removed by distillation. The residue was crystallized and washed with hexane-ether to obtain 5,5-dimethyl-3-(2-nitrobenzyl)thiazolidin-2-one (1.6 g).
Yield: 92%
Properties: melting point 127.8-129.2°C

11-4) Production of 3-(2-aminobenzyl)-4-methoxy-5,5-dimethylthiazolidin-2-one (Compound No. 2-54)

5,5-dimethyl-3-(2-nitrobenzyl)thiazolidin-2-one (1.4 g, 5.3 mmol), iron powder (1.5 g, 27 mmol), ammonium chloride (0.14 g, 2.7 mmol) were dissolved in ethanol (30 ml) and water (15 ml), and the solution was heated and refluxed for 1 hour. After cooled to room temperature, the reaction solution was filtered by suction and extracted with ethyl acetate. The ethyl acetate layer was washed with saturated sodium chloride solution, and then dried with anhydrous magnesium sulfate and the solvent was removed by distillation to obtain 3-(2-aminobenzyl)-5,5-dimethylthiazolidin-2-one (1.3 g).
Yield: 100%
Properties: $n_D$ 1.5788 (25°C)

11-5) Production of 5,5-dimethyl-3-[2-(trifluoromethanesulfonylamino)benzyl]thiazolidin-2-one (Compound No. 1-184)

3-(2-aminobenzyl)-5,5-dimethylthiazolidin-2-one (1.3 g, 5.3 mmol) and triethylamine (0.70 g, 6.9 mmol) were dissolved in chloroform (30 ml) and then the reaction solution was cooled to -40°C. To the reaction solution, trifluoromethanesulfonic anhydride (1.6 g, 5.8 mmol) was added dropwise, and the solution was stirred at -40°C for 30 min. The reaction solution was extracted with ethyl acetate after adding diluted hydrochloric acid. The ethyl acetate layer was washed with saturated sodium chloride solution, and then dried with anhydrous magnesium sulfate and the solvent was removed by distillation. The residue was purified by silica gel column chromatography (ethyl acetate:hexane = 1: 1) to obtain 5,5-dimethyl-3-[2-(trifluoromethanesulfonylamino)benzyl]thiazolidin-2-one (1.2 g).
Yield: 62%
Properties: melting point 115.0-117.5°C

Example 12. Production of 3-{2-[N-(isobutoxycarbonyl)-N-(trifluoromethanesulfonyl)amino]benzyl}-5,5-dimethylthiazolidin-2-one (Compound No. 1-185)

5,5-dimethyl-3-[2-(trifluoromethanesulfonylamino)benzyl]thiazolidin-2-one (0.25 g, 0.68 mmol) and sodium bicarbonate (0.11 g, 1.4 mmol) were suspended in acetonitrile (15 ml) and isobutyl chloroformate (0.19 g, 1.4 mmol) was added and refluxed for 2.5 hours. The reaction solution was cooled to room temperature and extracted with ethyl acetate after adding water. The ethyl acetate layer was washed with saturated sodium chloride solution, and then dried with anhydrous magnesium sulfate and the solvent was removed by distillation. The residue was purified by silica gel column

chromatography (ethyl acetate:hexane = 1:2) to obtain 3-{2-[N-(isobutoxycarbonyl)-N-(trifluoromethanesulfonyl)amino] benzyl}-5, 5-dimethylthiazolidin-2-one (0.28 g).
Yield: 88%
Properties: $n_D$ 1.4978 (26˚C)

Example 13. Production of 4-methoxy-5,5-dimethyl-3-[2-(trifluoromethanesulfonylamino)benzyl]oxazolidin-2-one (Compound No. 1-195)

13-1) Production of 5,5-dimethyl-3-(2-nitrobenzyl)oxazolidine-2,4-dione

A mixture of 5,5-dimethyloxazolidine-2,4-dione (1.9 g, 15 mmol), 2-nitrobenzylchloride (2.6 g, 15 mmol), potassium carbonate (2.5 g, 18 mmol) and N, N-dimethylformamide (15 ml) was heated at 80˚C for 2 hours while stirring. After cooling, the reaction mixture was mixed with water and extracted with ethyl acetate, and the organic layer was washed with water (twice), then with saturated sodium chloride solution and dried with anhydrous magnesium sulfate. After concentrated under reduced pressure, the organic layer was mixed with hexane, and precipitated solid material was collected by filtration to obtain 5,5-dimethyl-3-(2-nitrobenzyl)oxazolidine-2,4-dione (3.7 g).
Yield: 93%
Properties: melting point 133.5-134.5˚C

13-2) Production of 4-hydroxy-5,5-dimethyl-3-(2-nitrobenzyl)oxazolidin-2-one

5,5-dimethyl-3-(2-nitrobenzyl)oxazolidine-2,4-dione (2.1 g, 8.0 mmol) was dissolved in tetrahydrofuran (40 ml) and lithium borohydride (0.22 g, 10 mmol) was added to the reaction solution cooled on ice. The reaction mixture was stirred at room temperature overnight. Water was added to the reaction mixture, which was extracted with ethyl acetate. After being washed with water, and then saturated sodium chloride solution, the organic layer was dried with anhydrous magnesium sulfate. After the organic layer was concentrated under reduced pressure, the residue was purified by silica gel column chromatography (ethyl acetate:hexane = 1:1) to obtain 4-hydroxy-5,5-dimethyl-3-(2-nitrobenzyl)oxazolidin-2-one (1.9 g).
Yield: 88%
Properties: melting point 119-119.5˚C

13-3) Production of 4-methoxy-5,5-dimethyl-3-(2-nitrobenzyl)oxazolidin-2-one

To a methanol solution (35 ml) of 4-hydroxy-5,5-dimethyl-3-(2-nitrobenzyl)oxazolidin-2-one (1.9 g, 7.0 mmol), p-toluenesulfonic acid monohydrate (0.13 g, 0.70 mmol) was added and the mixture was refluxed for 4 hours while stirring. After cooling, the reaction mixture was concentrated under reduced pressure and then extracted with ethyl acetate. After being washed with saturated sodium bicarbonate solution, and then with saturated sodium chloride solution, the organic layer was dried with anhydrous magnesium sulfate. After the organic layer was concentrated under reduced pressure, the residue was purified by silica gel column chromatography (ethyl acetate:hexane = 1:2) to obtain 4-methoxy-5,5-dimethyl-3-(2-nitrobenzyl)oxazolidin-2-one (1.9 g).
Yield: 96%
Property: $^1$H-NMR [400 MHz, $CDCl_3$, $\delta$ (ppm)]: 8.01 (d, 1H), 7.61-7.68 (m, 2H), 7.46-7.50 (m, 1H), 4.83 (AB, 2H), 4.40 (s, 1H), 3.37 (s, 3H), 1.44 (s, 3H), 1.39 (s, 3H),

13-4) Production of 3-(2-aminobenzyl)-4-methoxy-5,5-dimethyloxazolidin-2-one (Compound No. 2-59)

A mixture of 4-methoxy-5,5-dimethyl-3-(2-nitrobenzyl)oxazolidin-2-one (1.4 g, 5.0 mmol), iron powder (1.4 g, 25 mmol), ammonium chloride (0.13 g, 2.5 mmol), ethanol (12 ml) and water (6 ml) was refluxed for 30 min while stirring. After cooling, tetrahydrofuran was added to reaction mixture and insoluble material was separated by filtration using celite. The filtrate was concentrated under reduced pressure and then extracted with ethyl acetate. After being washed with saturated sodium chloride solution, the organic layer was dried with anhydrous magnesium sulfate. After the organic layer was concentrated under reduced pressure, the residue was purified by silica gel column chromatography (ethyl acetate:hexane = 1:2) to obtain 3-(2-aminobenzyl)-4-methoxy-5,5-dimethyloxazolidin-2-one (1.2 g).
Yield: 95%
Property: $^1$H-NMR [400 MHz, $CDCl_3$,$\delta$ (ppm)]: 7.14 (dd, 1H), 7.05 (d, 1H), 6.69 (dd, 1H), 6.68 (d, 1H), 4.75 (d, 1H), 4.13 (s, 1H), 4.12 (brs, 2H), 4.07 (d, 1H), 3.38 (s, 3H), 1.40 (s, 3H), 1.27 (s, 3H),

13-5) Production of 4-methoxy-5,5-dimethyl-3-[2-(trifluoromethanesulfonylamino)benzyl]oxazolidin-2-one

(Compound No. 1-195)

3-(2-aminobenzyl)-4-methoxy-5,5-dimethyloxazolidin-2-one (0.88 g, 3.5 mmol) and triethylamine (0.43 g, 4.2 mmol) were dissolved in chloroform (35 ml) and cooled to -30˚C. Chloroform solution of trifluoromethane sulfonic anhydride (1.2 g, 4.2 mmol) was added dropwise (the reaction temperature: from -30 to -25˚C) and after that the stirring was continued for 30 min at the same temperature. Water was added to the reaction mixture while stirring and temperature was allowed to rise to room temperature. The organic layer was washed with water and then dried with anhydrous magnesium sulfate. After the organic layer was concentrated under reduced pressure, the residue was purified by silica gel column chromatography (ethyl acetate:hexane = 1:1) to obtain 4-methoxy-5,5-dimethyl-3-[2-(trifluoromethanesulfonylamino)benzyl]oxazolidin-2-one (1.2 g).
Yield: 90%
Property: $^1$H-NMR [400 MHz, $CDCl_3$, δ (ppm)]: 10.0 (brs, 1H), 7.58(d, 1H), 7.25-7.41 (m, 3H), 4.51 (s, 1H), 4.42 (AB, 2H), 3.51 (s, 3H), 1.42 (s, 3H), 1.36 (s, 3H)

Example 14. Production of 3-{2-[N-(isobutoxycarbonyl)-N-(trifluoromethanesufonyl)amino]benzyl}-4-methoxy-5,5-dimethyloxazolidin-2-one (Compound No. 1-197)

Isobutyl chloroformate (0.27 g, 2.0 mmol) was added to a mixture of 4-methoxy-5,5-dimethyl-3-[2-(trifluoromethanesulfonylamino)benzyl]oxazolidin-2-one (0.38 g, 1.0 mmol), sodium bicarbonate (0.17 g, 2.0 mmol) and acetonitrile (10 ml) and the reaction mixture was refluxed for 2 hours while stirring. After cooling, the reaction mixture was mixed with water and extracted with ethyl acetate, and the organic layer was washed with saturated sodium bicarbonate solution and then with saturated sodium chloride solution, and dried with anhydrous magnesium sulfate. After the organic layer was concentrated under reduced pressure, the residue was purified by silica gel column chromatography (ethyl acetate: hexane = 1:3) to obtain 3-{2-[N-(isobutoxycarbonyl)-N-(trifluoromethanesufonyl)amino]benzyl}-4-methoxy-5,5-dimethyloxazolidin-2-one (0.33 g).
Yield: 68%
Properties: $n_D$ 1.4794 (26˚C)

Example 15. Production of 4-hydroxy-5,5-dimethyl-3-[2-(trifluoromethanesulfonylamino)benzyl]oxazolidin-2-one (Compound No. 1-194)

15-1) 3-(2-aminobenzyl)-4-hydroxy-5,5-dimethyloxazolidin-2-one (Compound No. 2-64)

A mixture of 4-hydroxy-5,5-dimethyl-3-(2-nitrobenzyl)oxazolidin-2-one (1.1 g, 4.0 mmol), iron powder (1.1 g, 20 mmol), ammonium chloride (0.11g, 2.0 mmol), ethanol (10 ml) and water (5 ml) was heated and refluxed for 1 hour while stirring. After cooling, tetrahydrofuran was added to the reaction mixture and insoluble material was removed by filtration using celite. The filtrate was concentrated under reduced pressure and then extracted with ethyl acetate, and the organic layer was washed with saturated sodium chloride solution, and dried with anhydrous magnesium sulfate. The organic layer was concentrated under reduced pressure, and then hexane was added and precipitated solid material was collected by filtration to obtain 3-(2-aminobenzyl)-4-hydroxy-5,5-dimethyloxazolidin-2-one (0.90 g).
Yield: 95%
Properties: melting point 159-160˚C

15-2) Production of 4-hydroxy-5,5-dimethyl-3-[2-(trifluoromethanesulfonylamino)benzyl]oxazolidin-2-one

(Compound No. 1-194)

3-(2-aminobenzyl)-4-hydroxy-5,5-dimethyloxazolidin-2-one (0.35 g, 1.5 mmol) and triethylamine (0.18 g, 1.8 mmol) were dissolved in a mixed solvent of chloroform (15 ml) and tetrahydrofuran (7.5 ml) and cooled to -40˚C. Chloroform solution of trifluoromethane sulfonic anhydride (0.51 g, 1.8 mmol) was added dropwise (the reaction temperature: from -40 to -35˚C) and after that the stirring was continued for 30 min at the same temperature. Water was added to the reaction mixture while stirring and temperature was allowed to rise to room temperature. The organic layer was washed with water and then dried with anhydrous magnesium sulfate. After the organic layer was concentrated under reduced pressure, the residue was purified by silica gel column chromatography (ethyl acetate:hexane = 1:1) to obtain 4-hydroxy-5,5-dimethyl-3-[2-(trifluoromethanesulfonylamino)benzyl]oxazolidin-2-one (0.44 g).
Yield: 80%

Properties: melting point 88-92˚C

Example 16. Production of 5,5-dimethyl-3-[2-(trifluoromethanesulfonylamino)benzyl]oxazolidine-2,4-dione (Compound No. 1-187)

16-1) Production of 3-(2-aminobenzyl)-5,5-dimethyloxazolidine-2,4-dione (Compound No. 2-56)

A mixture of 5,5-dimethyl-3-(2-nitrobenzyl)oxazolidine-2,4-dione (3.2 g, 12 mmol), iron powder (3.4 g, 60 mmol), ammonium chloride (0.32g, 6.0 mmol), ethanol (30 ml) and water (15 ml) was heated and refluxed for 1 hour while stirring. After cooling, tetrahydrofuran was added to the reaction mixture and insoluble material was removed by filtration using celite. The filtrate was concentrated under reduced pressure and then extracted with ethyl acetate, and the organic layer was washed with saturated sodium chloride solution, and dried with anhydrous magnesium sulfate. The organic layer was concentrated under reduced pressure, and then hexane was added and precipitated solid material was collected by filtration to obtain 3-(2-aminobenzyl)-5,5-dimethyloxazolidine-2,4-dione (2.7 g).
Yield: 96%
Properties: melting point 122.5-123.5˚C

16-2) Production of 5,5-dimethyl-3-[2-(trifluoromethanesulfonylamino)benzyl]oxazolidine-2,4-dione (Compound No. 1-187)

3-(2-aminobenzyl)-5,5-dimethyloxazolidine-2,4-dione (1.4 g, 6.0 mmol) and triethylamine (0.73 g, 7.2 mmol) were dissolved in chloroform (40 ml) and cooled to -30˚C. Chloroform solution of trifluoromethane sulfonic anhydride (2.0 g, 7.2 mmol) was added dropwise (the reaction temperature: from -30 to -25˚C) and after that the stirring was continued for 30 min at the same temperature. Water was added to the reaction mixture while stirring and temperature was allowed to rise to room temperature. The organic layer was washed with water and then dried with anhydrous magnesium sulfate. After the organic layer was concentrated under reduced pressure, the residue was purified by silica gel column chromatography (ethyl acetate:hexane = 1:3) to obtain 5,5-dimethyl-3-[2-(trifluoromethanesulfonylamino) benzyl]oxazolidine-2,4-dione (2.2 g).
Yield: quantitative
Property: $^1$H-NMR [400 MHz, $CDCl_3$, δ (ppm)]: 9.30 (brs, 1H), 7.56 (d, 1H), 7.53 (d, 1H), 7.43 (dd, 1H), 7.32 (dd, 1H), 4.74 (s, 2H), 1.56 (s, 6H),

Example 17. Production of 3-{2-[N-(isobutoxycarbonyl)-N-(trifluoromethanesulfonyl)amino]benzyl}-5,5-dimethyloxazolidine-2,4-dione (Compound No. 1-189)

Isobutyl chloroformate (0.41 g, 3.0 mmol) was added to a mixture of 5,5-dimethyl-3-[2-(trifluoromethanesulfonylamino)benzyl]oxazolidine-2,4-dione (0.55 g, 1.5 mmol), sodium bicarbonate (0.25 g, 3.0 mmol) and acetonitrile (10 ml) and the reaction mixture was heated and refluxed for 2 hours while stirring. After cooling, the reaction mixture was mixed with water and extracted with ethyl acetate, and the organic layer was washed with saturated sodium bicarbonate solution and then with saturated sodium chloride solution, and dried with anhydrous magnesium sulfate. After the organic layer was concentrated under reduced pressure, the residue was purified by silica gel column chromatography (ethyl acetate:hexane = 1:5) to obtain 3-{2-[N-(isobutoxycarbonyl)-N-(trifluoromethanesulfonyl)amino]benzyl}-5,5-dimethyloxazolidine-2,4-dione (0.60 g).
Yield: 86%
Properties: $n_D$ 1.4867 (15˚C)
The followings are representative formulation examples and test examples of the present invention but the present invention is not limited to those. In formulation examples "parts" represents "weight parts". Formulation Example 1. Emulsion

| | |
|---|---|
| Compound of the present invention | 10 parts |
| Xylene | 70 parts |
| N-methyl pyrrolidone 10 | parts |
| Mixture of polyoxyethylenenonylphenyl ether and calcium alkylbenzenesulfonate | 10 parts |

Above components are homogeneously mixed and dissolved to prepare emulsion.
Formulation Example 2. Powder

| | |
|---|---|
| Compound of the present invention | 3 parts |
| Clay powder | 82 parts |
| Diatom powder | 15 parts |

Above components are homogenously mixed and pulverized to prepare powder.

Formulation Example 3. Granules

| | |
|---|---|
| Compound of the present invention | 5 parts |
| Mixed powder of bentonite and clay | 90 parts |
| Calcium ligninsulfonate | 5 parts |

Above components are homogeneously mixed, kneaded with appropriate amount of water, granulated and dried to prepare granules.

Formulation Example 4. Water dispersible powder

| | |
|---|---|
| Compound of the present invention | 20 parts |
| Kaolin and synthetic high dispersion silicate | 75 parts |
| Mixture of polyoxyethylenenonylphenyl ether and calcium alkylbenzenesulfonate | 5 parts |

Above components are homogenously mixed and pulverized to prepare water dispersible powder.

Test Example 1. Herbicidal efficacy on rice paddy weeds pre-emergence

A 75 $cm^2$ plastic pot was filled with soil (clay loam) and planted seeds of bulrush which is a rice paddy weed. After covering the top with 75 $cm^3$ soil mixed with seeds of false pimpernel, the pot was filled with water so that the soil submerged under 5 cm depth of water. The predetermined effective dosage (1000 g/ha or 300 g/ha as an active ingredient) of a formulation containing the compound of the present invention prepared according to Formulation Example 1-4 as an active ingredient was diluted in water and added to the pot water surface dropwise. The pot was kept in a hot house for 21 days and the herbicidal efficacy was investigated. The herbicidal efficacy was evaluated according to the following standard by comparing with untreated control. Results are shown in Table 6. "-" in the table represents test not done.

The standard of judgment for herbicidal efficacy (degree of growth inhibition) and phytotoxicity 4 ... 90%-100% of herbicidal efficacy or phytotoxicity 3 ... 70%-89% of herbicidal efficacy or phytotoxicity 2 ... 40%-69% of herbicidal efficacy or phytotoxicity 1 ... 1%-39% of herbicidal efficacy or phytotoxicity 0 ... 0% of herbicidal efficacy or phytotoxicity

Test Example 2. Herbicidal efficacy on rice paddy weeds post-emergence

A 75 $cm^2$ plastic pot was filled with soil (clay loam) and planted seeds of barnyard glass and bulrush which is a rice paddy weed. After covering the top with 75 $cm^3$ soil mixed with seeds of false pimpernel, the pot was filled with water so that the soil submerged under 5 cm depth of water and kept in a hot house. The test plants, at the one leaf stage, were treated with the solution of a formulation containing the compound of the present invention as an active ingredient at the predetermined effective dosage (1000 g/ha or 300 g/ha as an active ingredient). The pot was kept in a hot house for 21 days after the treatment and the herbicidal efficacy was investigated and evaluated according to the standard in Test Example 1 by comparing with the untreated control. Results are shown in Table 6. "-" in the table represents test not done.

Test Example 3. Phytotoxicity test for transplanted paddy rice plant

A 75 $cm^2$ plastic pot was filled with soil (clay loam), and water was added so that the soil was submerged under 5 cm depth of water. Two paddy rice plant (cultivar: Nihonbare) at two leaves stage were transplanted at the depth of 1 cm and kept in a hot house. After 5 days of transplantation, the plants were treated with the solution of a formulation containing the compound of the present invention as an active ingredient at the predetermined effective dosage (1000 g/ha as an active ingredient). The pot was kept in a hot house for 21 days after the treatment and the phytotoxicity was investigated and evaluated according to the standard in Test Example 1 by comparing with the untreated control. Results are shown in Table 6. "-" in the table represents test not done.

[Table 6]

Table 6

| Compound No. | Dosage g/ha | Burnyard grass post | Bulrush pre | Bulrush post | False pimpernel pre | False pimpernel post | Phytotoxicity to paddy rice |
|---|---|---|---|---|---|---|---|
| 1-1 | 1000 | 4 | 4 | 4 | 4 | 4 | 1 |
| 1-2 | 1000 | 4 | 4 | 4 | 4 | 4 | 1 |
| 1-3 | 1000 | 4 | 4 | 4 | 4 | 4 | 4 |
| 1-4 | 1000 | 4 | 4 | 4 | 4 | 4 | 2 |
| 1-5 | 1000 | 4 | 4 | 4 | 4 | 4 | 2 |
| 1-6 | 1000 | 4 | 4 | 4 | 4 | 4 | 1 |
| 1-7 | 300 | 1 | 4 | 4 | 4 | 2 | 1 |
| 1-8 | 300 | 4 | 4 | 4 | 4 | 2 | 0 |
| 1-9 | 300 | 4 | 4 | 4 | 4 | 2 | 2 |
| 1-10 | 1000 | 4 | 4 | 4 | 4 | 4 | 1 |
| 1-11 | 300 | 0 | 4 | 3 | 2 | 1 | 0 |
| 1-12 | 300 | 2 | 4 | 4 | 2 | 2 | 0 |
| 1-13 | - | - | - | - | - | - | - |
| 1-14 | - | - | - | - | - | - | - |
| 1-15 | 1000 | 4 | 4 | 4 | 4 | 4 | 1 |
| 1-16 | 1000 | 4 | 4 | 4 | 4 | 4 | 0 |
| 1-17 | 1000 | 4 | 4 | 4 | 4 | 4 | 0 |
| 1-18 | 1000 | 4 | 4 | 4 | 4 | 4 | 0 |
| 1-19 | 300 | 2 | 4 | 4 | 3 | 2 | 0 |
| 1-20 | 300 | 4 | 4 | 4 | 3 | 3 | 0 |
| 1-21 | 1000 | 4 | 4 | 4 | 4 | 3 | 0 |
| 1-22 | 1000 | 4 | 4 | 4 | 4 | 4 | 0 |
| 1-23 | 1000 | 4 | 4 | 4 | 4 | 4 | 1 |
| 1-24 | 1000 | 4 | 4 | 4 | 4 | 4 | 0 |
| 1-25 | - | - | - | - | - | - | - |
| 1-26 | - | - | - | - | - | - | - |
| 1-27 | - | - | - | - | - | - | - |
| 1-28 | - | - | - | - | - | - | - |
| 1-29 | 1000 | 4 | 4 | 4 | 4 | 4 | 2 |
| 1-30 | 1000 | 4 | 4 | 4 | 4 | 4 | 0 |
| 1-31 | 1000 | 4 | 4 | 4 | 4 | 4 | 1 |
| 1-32 | 1000 | 4 | 4 | 4 | 4 | 4 | 0 |
| 1-33 | 1000 | 4 | 4 | 4 | 4 | 4 | 2 |
| 1-34 | 1000 | 4 | 4 | 4 | 4 | 4 | 1 |
| 1-35 | 1000 | - | 4 | 4 | 4 | 4 | 1 |
| 1-36 | 1000 | 4 | 4 | 4 | 4 | 4 | 1 |
| 1-37 | 300 | 0 | 0 | 0 | 1 | 3 | 0 |
| 1-38 | 300 | 1 | 3 | 3 | 2 | 1 | 0 |
| 1-39 | 1000 | 2 | 4 | 4 | 4 | 4 | 0 |
| 1-40 | 1000 | 4 | 4 | 4 | 4 | 4 | 0 |
| 1-41 | 1000 | 4 | 4 | 3 | 4 | 2 | 1 |
| 1-42 | 1000 | 4 | 3 | 3 | 4 | 3 | 0 |
| 1-43 | 1000 | 4 | 4 | 4 | 4 | 4 | 2 |
| 1-44 | 1000 | 4 | 4 | 4 | 4 | 4 | 2 |
| 1-45 | 1000 | 4 | 4 | 4 | 4 | 4 | 2 |
| 1-46 | 1000 | 4 | 4 | 4 | 4 | 4 | 1 |
| 1-47 | 1000 | 4 | 4 | 4 | 4 | 4 | 2 |
| 1-48 | 1000 | 4 | 4 | 4 | 4 | 4 | 1 |

(continued)

| Compound No. | Dosage g/ha | Burnyard grass post | Bulrush pre | Bulrush post | False pimpernel pre | False pimpernel post | Phytotoxicity to paddy rice |
|---|---|---|---|---|---|---|---|
| 1-49 | 1000 | 4 | 4 | 4 | 4 | 4 | 2 |
| 1-50 | 1000 | 4 | 4 | 4 | 4 | 4 | 2 |
| 1-51 | 300 | 0 | 3 | 4 | 4 | 3 | 0 |
| 1-52 | 300 | 3 | 4 | 4 | 4 | 4 | 0 |
| 1-53 | 1000 | 4 | 4 | 4 | 4 | 4 | 0 |
| 1-54 | 1000 | 4 | 4 | 4 | 4 | - | 0 |
| 1-55 | 1000 | 4 | 4 | 4 | 4 | 4 | 1 |
| 1-56 | 300 | 4 | 4 | 4 | 4 | 3 | 1 |
| 1-57 | 1000 | 4 | 4 | 4 | 4 | 4 | 1 |
| 1-58 | 1000 | 4 | 4 | 4 | 4 | 4 | 1 |
| 1-59 | 300 | 3 | 4 | 4 | 4 | 4 | 3 |
| 1-60 | 1000 | 4 | 4 | 4 | 4 | 4 | 3 |
| 1-61 | 1000 | 4 | 4 | 4 | 4 | 4 | 1 |
| 1-62 | 1000 | 4 | 4 | 4 | 4 | 4 | 1 |
| 1-63 | 1000 | 4 | 4 | 4 | 4 | 4 | 2 |
| 1-64 | 1000 | 4 | 4 | 4 | 4 | 4 | 2 |
| 1-65 | 1000 | 4 | 4 | 4 | 4 | 4 | 1 |
| 1-66 | 1000 | 4 | 4 | 4 | 4 | 4 | 1 |
| 1-67 | 1000 | 4 | 4 | 4 | 4 | 4 | 1 |
| 1-68 | 1000 | 4 | 4 | 4 | 4 | 4 | 1 |
| 1-69 | 300 | 3 | 4 | 4 | 4 | 4 | 1 |
| 1-70 | 1000 | 4 | 4 | 4 | 4 | 4 | 1 |
| 1-71 | 1000 | 4 | 4 | 4 | 4 | 4 | 1 |
| 1-72 | 1000 | 4 | 4 | 4 | 4 | 4 | 1 |
| 1-73 | 1000 | 4 | 4 | 4 | 4 | 4 | 4 |
| 1-74 | 1000 | 4 | 4 | 4 | 4 | 4 | 4 |
| 1-75 | 1000 | 4 | 4 | 4 | 4 | 4 | 1 |
| 1-76 | 1000 | 4 | 4 | 4 | 4 | 4 | 2 |
| 1-77 | 1000 | 4 | 4 | 4 | 4 | 4 | 1 |
| 1-78 | 1000 | 4 | 4 | 4 | 4 | 4 | 1 |
| 1-79 | 1000 | 4 | 4 | 4 | 4 | 4 | 2 |
| 1-80 | 1000 | 4 | 4 | 4 | 4 | 4 | 2 |
| 1-81 | 1000 | 4 | 4 | 4 | 4 | 4 | 2 |
| 1-82 | 1000 | 4 | 4 | 4 | 4 | 4 | 2 |
| 1-83 | 300 | - | 4 | 4 | 1 | 1 | 0 |
| 1-84 | 300 | - | 4 | 3 | 4 | 4 | 1 |
| 1-85 | 1000 | 4 | 4 | 4 | 4 | 4 | 2 |
| 1-86 | 1000 | 4 | 4 | 4 | 4 | 4 | 1 |
| 1-87 | 300 | - | 4 | 4 | 4 | 4 | 1 |
| 1-88 | 300 | - | 4 | 4 | 4 | 4 | 1 |
| 1-89 | 1000 | 4 | 4 | 4 | 4 | 4 | 1 |
| 1-90 | 1000 | 4 | 4 | 4 | 4 | 4 | 3 |
| 1-91 | 1000 | 4 | 4 | 4 | 4 | 4 | 3 |
| 1-92 | 1000 | 4 | 4 | 4 | 4 | 4 | 3 |
| 1-93 | 1000 | 4 | 4 | 4 | 4 | 4 | 1 |
| 1-94 | 1000 | 4 | 4 | 4 | 4 | 4 | 1 |
| 1-95 | 1000 | 4 | 4 | 4 | 4 | 4 | 3 |
| 1-96 | 1000 | 4 | 4 | 4 | 4 | 4 | 3 |

(continued)

| Compound No. | Dosage g/ha | Burnyard grass post | Bulrush pre | Bulrush post | False pimpernel pre | False pimpernel post | Phytotoxicity to paddy rice |
|---|---|---|---|---|---|---|---|
| 1-97 | 1000 | 4 | 4 | 4 | 4 | 4 | 1 |
| 1-98 | 1000 | 4 | 4 | 4 | 4 | 4 | 1 |
| 1-99 | 1000 | 4 | 4 | 4 | 4 | 4 | 1 |
| 1-100 | 1000 | 4 | 4 | 4 | 4 | 4 | 1 |
| 1-101 | 1000 | 4 | 4 | 4 | 4 | 4 | 1 |
| 1-102 | 1000 | 4 | 4 | 4 | 4 | 4 | 1 |
| 1-103 | 300 | 4 | 4 | 4 | 4 | 4 | 2 |
| 1-104 | 1000 | 4 | 4 | 4 | 4 | 4 | 2 |
| 1-105 | 1000 | 4 | 4 | 4 | 4 | 4 | 1 |
| 1-106 | 300 | 0 | 4 | 4 | 2 | 2 | 0 |
| 1-107 | 300 | 3 | 4 | 4 | 4 | 1 | 0 |
| 1-108 | 1000 | 4 | 4 | 4 | 4 | 4 | 2 |
| 1-109 | 1000 | - | 4 | 4 | 4 | 4 | 1 |
| 1-110 | 1000 | 4 | 4 | 4 | 4 | 4 | 2 |
| 1-111 | 1000 | 4 | 4 | 4 | 4 | 4 | 2 |
| 1-112 | 1000 | 4 | 4 | 4 | 4 | 4 | 2 |
| 1-113 | 1000 | 4 | 4 | 4 | 4 | 4 | 1 |
| 1-114 | 1000 | 4 | 4 | 4 | 4 | 4 | 2 |
| 1-115 | 1000 | 4 | 4 | 4 | 4 | 4 | 3 |
| 1-116 | 1000 | 4 | 4 | 4 | 4 | 4 | 1 |
| 1-117 | 1000 | 4 | 4 | 4 | 4 | 4 | 2 |
| 1-118. | 1000 | 4 | 4 | 4 | 4 | 4 | 1 |
| 1-119 | 1000 | 4 | 4 | 4 | 4 | 4 | 1 |
| 1-120 | 300 | 3 | 4 | 4 | 4 | 4 | 2 |
| 1-121 | 300 | 1 | 4 | 4 | 4 | 4 | 1 |
| 1-122 | 1000 | 4 | 4 | 4 | 4 | 4 | 1 |
| 1-123 | 1000 | 4 | 4 | 4 | 4 | 4 | 1 |
| 1-124 | 1000 | 4 | 4 | 4 | 4 | 4 | 1 |
| 1-125 | 1000 | 4 | 4 | 4 | 4 | 4 | 1 |
| 1-126 | 1000 | 4 | 4 | 4 | 4 | 4 | 2 |
| 1-127 | 1000 | 4 | 4 | 4 | 4 | 4 | 1 |
| 1-128 | 100 | 3 | 2 | 3 | 4 | 2 | 1 |
| 1-129 | 100 | 3 | 2 | 4 | 4 | 3 | 1 |
| 1-130 | 1000 | 4 | 4 | 4 | 4 | 4 | 1 |
| 1-131 | 1000 | 4 | 4 | 4 | 4 | 4 | 2 |
| 1-132 | 1000 | 4 | 4 | 4 | 4 | 4 | 1 |
| 1-133 | 1000 | 4 | 4 | 4 | 4 | 4 | 1 |
| 1-134 | 1000 | 4 | 4 | 4 | 4 | 4 | 1 |
| 1-135 | 300 | 2 | 4 | 4 | 4 | 4 | 1 |
| 1-136 | 1000 | 4 | 4 | 4 | 4 | 4 | 1 |
| 1-137 | 300 | 4 | 4 | 4 | 4 | 4 | 2 |
| 1-138 | 1000 | 4 | 4 | 4 | 4 | 4 | 2 |
| 1-139 | 300 | 3 | 4 | 4 | 4 | 4 | 3 |
| 1-140 | 300 | 2 | 4 | 4 | 4 | 4 | 1 |
| 1-141 | 300 | 5 | 3 | 4 | 4 | 4 | 1 |
| 1-142 | 1000 | 4 | 4 | 4 | 4 | 4 | 1 |
| 1-143 | 1000 | 4 | 4 | 4 | 4 | 4 | 1 |
| 1-144 | 1000 | 4 | 4 | 4 | 4 | 4 | 1 |

(continued)

| Compound No. | Dosage g/ha | Burnyard grass post | Bulrush pre | Bulrush post | False pimpernel pre | False pimpernel post | Phytotoxicity to paddy rice |
|---|---|---|---|---|---|---|---|
| 1-145 | 1000 | 4 | 4 | 4 | 4 | 4 | 2 |
| 1-146 | 1000 | 4 | 4 | 4 | 4 | 4 | 1 |
| 1-147 | 300 | - | 4 | 4 | 4 | 4 | 1 |
| 1-148 | 1000 | 4 | 4 | 4 | 4 | 4 | 3 |
| 1-149 | 1000 | 4 | 4 | 4 | 4 | 4 | 2 |
| 1-150 | 1000 | 4 | 4 | 4 | 4 | 4 | 2 |
| 1-151 | 1000 | - | 4 | 4 | 4 | 4 | 1 |
| 1-152 | 1000 | 4 | 4 | 4 | 4 | 4 | 1 |
| 1-153 | 1000 | 4 | 4 | 4 | 4 | 4 | 1 |
| 1-154 | 300 | 1 | 4 | 4 | 4 | 4 | 1 |
| 1-155 | 300 | 1 | 4 | 4 | 4 | 4 | 1 |
| 1-156 | 300 | - | 4 | 4 | 4 | 4 | 1 |
| 1-157 | 1000 | 4 | 4 | 4 | 4 | 4 | 1 |
| 1-158 | 1000 | 4 | 4 | 4 | 4 | 4 | 1 |
| 1-159 | 1000 | 4 | 4 | 4 | 4 | 4 | 1 |
| 1-160 | 300 | 4 | 4 | 4 | 4 | 4 | 1 |
| 1-161 | 1000 | 4 | 4 | 4 | 4 | 4 | 1 |
| 1-162 | 1000 | 4 | 4 | 4 | 4 | 4 | 1 |
| 1-163 | - | - | - | - | - | - | - |
| 1-164 | 1000 | - | 4 | 4 | 4 | 4 | 1 |
| 1-165 | 1000 | - | 4 | 4 | 4 | 4 | 1 |
| 1-166 | 1000 | - | 4 | 4 | 4 | 4 | 2 |
| 1-167 | 1000 | 4 | 4 | 4 | 4 | 4 | 1 |
| 1-168 | 1000 | 4 | 4 | 4 | 4 | 4 | 1 |
| 1-169 | 1000 | 4 | 4 | 4 | 4 | 4 | 1 |
| 1-170 | 1000 | - | 4 | 4 | 4 | 4 | 2 |
| 1-171 | 1000 | 4 | 4 | 4 | 4 | 4 | 1 |
| 1-172 | 1000 | - | 4 | 4 | 4 | 4 | 1 |
| 1-173 | 1000 | - | 4 | 4 | 4 | 4 | 1 |
| 1-174 | 1000 | 4 | 4 | 4 | 4 | 4 | 1 |
| 1-175 | 1000 | 4 | 4 | 4 | 4 | 4 | 1 |
| 1-176 | 1000 | - | 4 | 4 | 4 | 4 | 1 |
| 1-177 | 1000 | 4 | 4 | 4 | 4 | 4 | 1 |
| 1-178 | 1000 | 4 | 4 | 4 | 4 | 4 | 1 |
| 1-179 | 1000 | 4 | 4 | 4 | 4 | 4 | 0 |
| 1-180 | 1000 | 4 | 4 | 4 | 4 | 4 | 2 |
| 1-181 | 1000 | 4 | 4 | 4 | 4 | 4 | 1 |
| 1-182 | 1000 | 4 | 4 | 4 | 4 | 4 | 1 |
| 1-183 | 1000 | 4 | 4 | 4 | 4 | 4 | 1 |
| 1-184 | 1000 | 4 | 4 | 4 | 4 | 4 | 1 |
| 1-185 | 1000 | 4 | 4 | 4 | 4 | 4 | 0 |
| 1-186 | 1000 | 4 | 4 | 4 | 4 | 4 | 1 |
| 1-187 | 1000 | 4 | 4 | 4 | 4 | 4 | 3 |
| 1-188 | 1000 | 4 | 4 | 4 | 4 | 4 | 1 |
| 1-189 | 1000 | 4 | 4 | 4 | 4 | 4 | 1 |
| 1-190 | 300 | - | 4 | 4 | 4 | 2 | 0 |
| 1-191 | 300 | - | 4 | 4 | 4 | 2 | 0 |
| 1-192 | 300 | 0 | 4 | 4 | 4 | 0 | 1 |

(continued)

| Compound No. | Dosage g/ha | Burnyard grass post | Bulrush pre | Bulrush post | False pimpernel pre | False pimpernel post | Phytotoxicity to paddy rice |
|---|---|---|---|---|---|---|---|
| 1-193 | 300 | - | 4 | 4 | 4 | 1 | 0 |
| 1-194 | - | - | - | - | - | - | - |
| 1-195 | 300 | 1 | 4 | 4 | 1 | 3 | 2 |
| 1-196 | 1000 | 4 | 4 | 4 | 4 | 4 | 1 |
| 1-197 | 300 | 4 | 4 | 4 | 3 | 1 | 2 |
| 1-198 | 300 | - | 4 | - | - | - | 2 |
| 1-199 | - | - | - | - | - | - | - |
| 1-200 | 1000 | 1 | 4 | 3 | 2 | 3 | 0 |
| 1-201 | - | - | - | - | - | - | - |
| 1-202 | - | - | - | - | - | - | - |
| 1-203 | - | - | - | - | - | - | - |
| 1-204 | - | - | - | - | - | - | - |
| 1-205 | - | - | - | - | - | - | - |
| 1-206 | - | - | - | - | - | - | - |
| 1-207 | 300 | 0 | 4 | 4 | 4 | 1 | 1 |
| 1-208 | 300 | - | 4 | 4 | 4 | 1 | 0 |
| 1-209 | 300 | - | 3 | 3 | 4 | 2 | 1 |
| 1-210 | 300 | - | 3 | 4 | 4 | 0 | 0 |
| 1-211 | 1000 | 4 | 4 | 4 | - | - | 4 |
| 1-212 | 1000 | 3 | 4 | 2 | 1 | 2 | 3 |
| 1-213 | 1000 | 5 | 4 | 4 | 5 | 2 | 1 |
| 1-214 | 1000 | 0 | 1 | 1 | 5 | 5 | 0 |
| 1-215 | 1000 | 1 | 2 | 2 | 4 | 3 | 0 |
| 1-216 | - | - | - | - | - | - | - |
| 1-217 | - | - | - | - | - | - | - |

Test Example 4. Low dosage test for rice paddy weeds

A 75 $cm^2$ plastic pot was filled with soil (clay loam) and planted seeds of bulrush which is a rice paddy weed. After covering the top with 75 $cm^3$ soil, the pot was filled with water so that the soil submerged under 5 cm depth of water and kept in a hot house. The test plants, at the one leaf stage, were treated with the solution of a formulation containing the compound of the present invention as an active ingredient at the predetermined effective dosage (300 g/ha as an active ingredient). The pots were kept in a hot house for 21 days after the treatment and the herbicidal efficacy was investigated and evaluated according to the standard in Test Example 1 by comparing with the untreated control. Results are shown in Table 7. Further, as a comparative compound, the compound No. 6-28 described in the JP-A-2004-107322 was used.

Test Example 5. Selectivity between transplanted paddy rice plant and weeds

Phytotoxicity test for transplanted paddy rice plants was carried out, as in Test Example 3, by the treatment of 3000, 1000, 300 and 100 (g/ha of the active ingredient) dosage, and herbicidal test to weed post-emergence was carried out to Burlush seeded as in Test Example 2 treating at 300, 100, 30 and 10 (g/ha) dosage. The pots were kept in a hot house for 21 days after the treatment, and the phytotoxicity and the herbicidal efficacy was investigated and evaluated according to the standard in Test Example 1 by comparing with the untreated control. From these results, selective indices were calculated according to the following formula, and the selectivity between transplanted paddy rice plant and Bulrush was evaluated. Results are shown in Table 7. Further, as a comparative compound, the compound No. 6-28 described in the JP-A-2004-107322 was used.

Selectivity Index = (The highest dosage at which no phytotoxicity to paddy rice was seen) / (The lowest dosage at which effective level 4 was demonstrated)

[Table 7]

Table 7

| Compound No. | Herbicide effect to Bulrush | Selective Index |
|---|---|---|
| 1-2 | 3 | 3 |
| 1-3 | 4 | 3.3 |
| 1-4 | 4 | 10 |
| 1-5 | 4 | ≧10 |
| 1-6 | 3 | 3 |
| 1-15 | 4 | 10 |
| 1-16 | 4 | 33 |
| 1-17 | 3 | 10 |
| 1-18 | 4 | 33 |
| 1-21 | 4 | 33 |
| 1-22 | 4 | 33 |
| 1-23 | 4 | 10 |
| 1-24 | 4 | 33 |
| 1-31 | 4 | ≧10 |
| 1-32 | 3 | 10 |
| 1-40 | 4 | 100 |
| 1-43 | 4 | 10 |
| 1-44 | 4 | 30 |
| 1-46 | 4 | 10 |
| 1-48 | 3 | 10 |
| 1-49 | 4 | 10 |
| 1-50 | 4 | 10 |
| 1-53 | 3 | 10 |
| 1-54 | 4 | 33.3 |
| 1-57 | 4 | 10 |
| 1-58 | 4 | ≧30 |
| 1-59 | 4 | 3.3 |
| 1-60 | 4 | ≧ 30 |
| 1-61 | 4 | 3 |
| 1-62 | 4 | 10 |
| 1-63 | 4 | ≧30 |
| 1-64 | 4 | ≧30 |
| 1-65 | 4 | 10 |
| 1-66 | 4 | ≧30 |
| 1-67 | 4 | ≧30 |
| 1-68 | 4 | 10 |
| 1-71 | 4 | 10 |
| 1-72 | 4 | 10 |
| 1-75 | 4 | ≧30 |
| 1-76 | 4 | 30 |

(continued)

| Compound No. | Herbicide effect to Bulrush | Selective Index |
|---|---|---|
| 1-77 | 4 | 10 |
| 1-78 | 4 | 10 |
| 1-79 | 4 | 10 |
| 1-80 | 4 | 30 |
| 1-81 | 4 | 30 |
| 1-82 | 4 | ≧30 |
| 1-85 | 4 | 10 |
| 1-86 | 4 | 10 |
| 1-87 | 3 | 3 |
| 1-89 | 4 | 10 |
| 1-90 | 4 | 30 |
| 1-91 | 4 | 30 |
| 1-92 | 4 | 30 |
| 1-93 | 4 | ≧30 |
| 1-94 | 4 | ≧30 |
| 1-95 | 4 | 30 |
| 1-97 | 4 | 30 |
| 1-98 | 4 | 10 |
| 1-99 | 4 | 10 |
| 1-100 | 4 | 10 |
| 1-101 | 4 | 10 |
| 1-102 | 4 | 10 |
| 1-105 | 4 | 10 |
| 1-108 | 4 | 30 |
| 1-109 | 4 | > 30 |
| 1-110 | 4 | ≧30 |
| 1-111 | 4 | ≧30 |
| 1-112 | 4 | 30 |
| 1-113 | 4 | 30 |
| 1-114 | 4 | 30 |
| 1-115 | 4 | 10 |
| 1-116 | 4 | 10 |
| 1-117 | 4 | 10 |
| 1-118 | 4 | 30 |
| 1-119 | 4 | 30 |
| 1-120 | 4 | 10 |
| 1-122 | 4 | 10 |
| 1-124 | 4 | 10 |
| 1-125 | 4 | 10 |
| 1-126 | 4 | 10 |
| 1-127 | 4 | 30 |
| 1-129 | 4 | 10 |
| 1-130 | 4 | 10 |
| 1-131 | 4 | 10 |
| 1-132 | 4 | 10 |
| 1-133 | 4 | 10 |
| 1-134 | 4 | 10 |
| 1-135 | 3 | 3 |
| 1-136 | 4 | 10 |
| 1-142 | 4 | ≧30 |

(continued)

| Compound No. | Herbicide effect to Bulrush | Selective Index |
|---|---|---|
| 1-143 | 4 | 10 |
| 1-144 | 4 | 10 |
| 1-145 | 4 | 10 |
| 1-146 | 4 | 10 |
| 1-147 | 4 | 10 |
| 1-148 | 4 | ≧30 |
| 1-149 | 4 | 10 |
| 1-150 | 4 | 10 |
| 1-151 | 3 | 3 |
| 1-152 | 4 | 10 |
| 1-153 | 4 | 10 |
| 1-157 | 3 | 3 |
| 1-167 | 4 | ≧10 |
| 1-168 | 4 | 10 |
| 1-169 | 4 | 10 |
| 1-170 | 4 | 10 |
| 1-171 | 4 | 10 |
| 1-172 | 4 | 10 |
| 1-178 | 4 | 10 |
| 1-179 | 4 | 33.3 |
| 1-181 | 4 | 10 |
| 1-183 | 4 | 10 |
| 1-184 | 4 | 10 |
| 1-185 | 4 | 33.3 |
| 1-186 | 4 | 10 |
| 1-187 | 4 | ≧10 |
| 1-188 | 4 | ≧10 |
| 1-189 | 4 | ≧10 |
| 1-192 | 4 | 10 |
| 1-196 | 4 | 10 |
| 1-197 | 4 | 10 |
| Compound for comparison | 2 | 1 |

Results in Table 7 indicate that the compound of the present invention has higher herbicidal efficacy than the compound for comparison and is safe to transplanted paddy rice plants.

## Claims

**1.** A haloalkyl sulfonanilide derivative represented by the general formula (I):

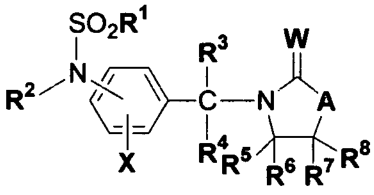

( I )

wherein $R^1$ is a halo ($C_1$-$C_6$) alkyl group;

$R^2$ is a hydrogen atom, a ($C_1$-$C_6$) alkoxycarbonyl ($C_1$-$C_6$) alkyl group, a ($C_1$-$C_{18}$) alkylcarbonyl group, a halo ($C_1$-$C_6$) alkylcarbonyl group, a phenylcarbonyl group, a substituted phenylcarbonyl group with 1 to 5 substituents which may be the same or different and are selected from Y (Y is described below), a ($C_1$-$C_{18}$) alkoxycarbonyl group, a ($C_2$-$C_{18}$) alkenyloxycarbonyl group, a ($C_2$-$C_{18}$) alkynyloxycarbonyl group, a halo ($C_1$-$C_6$) alkoxycarbonyl group, a ($C_1$-$C_6$) alkoxy ($C_1$-$C_6$) alkoxycarbonyl group, a ($C_1$-$C_6$) alkylthio ($C_1$-$C_6$) alkoxycarbonyl group, a ($C_1$-$C_6$) alkylsulfinyl ($C_1$-$C_6$) alkoxycarbonyl group, a ($C_1$-$C_6$) alkylsulfonyl ($C_1$-$C_6$) alkoxycarbonyl group, a phenoxycarbonyl group, a substituted phenoxycarbonyl group with 1 to 5 substituents which may be the same or different and are selected from Y (Y is described below), a phenoxy ($C_1$-$C_6$) alkylcarbonyl group, a substituted phenoxy ($C_1$-$C_6$) alkylcarbonyl group with 1 to 5 substituents which may be the same or different and are selected from Y (Y is described below), a benzyloxycarbonyl group, a substituted benzyloxycarbonyl group with 1 to 5 substituents which may be the same or different and are selected from Y (Y is described below), a ($C_1$-$C_6$) alkylthiocarbonyl group, a ($C_1$-$C_6$) alkylsulfonyl group, a halo ($C_1$-$C_6$) alkylsulfonyl group, phenylsulfonyl group, or a substituted phenylsulfonyl group with 1 to 5 substituent which may be the same or different and are selected from Y (Y is described below);

$R^3$ and $R^4$ may be the same or different, and each are a hydrogen atom, a ($C_1$-$C_6$) alkyl group, a ($C_3$-$C_6$) cycloalkyl group, a ($C_1$-$C_6$) alkoxy group, a halogen atom or a cyano group, and $R^3$ and $R^4$ may also be mutually bonded to form a 3- to 7-membered ring;

$R^5$, $R^6$, $R^7$ and $R^8$ may be the same or different and are a hydrogen atom, a halogen atom, a ($C_1$-$C_6$) alkyl group, a ($C_3$-$C_6$) cycloalkyl group, a ($C_1$-$C_6$) alkoxy group, a halo ($C_1$-$C_6$) alkyl group, a ($C_1$-$C_6$) alkoxy ($C_1$-$C_6$) alkyl group, a ($C_1$-$C_6$) alkylcarbonyloxy ($C_1$-$C_6$) alkyl group, a mono ($C_1$-$C_6$) alkylamino ($C_1$-$C_6$) alkyl group, a di ($C_1$-$C_6$) alkylamino ($C_1$-$C_6$) alkyl group in which the alkyl groups may be the same or different, a mono ($C_1$-$C_6$) alkylaminocarbonyl ($C_1$-$C_6$) alkyl group, a di ($C_1$-$C_6$) alkylaminocarbonyl ($C_1$-$C_6$) alkyl group in which the alkyl groups may be the same or different, a phenyl ($C_1$-$C_6$) alkyl group, a substituted phenyl ($C_1$-$C_6$) alkyl group with 1 to 5 substituents on the ring which may be the same or different and are selected from Y (Y is described below), a phenoxy ($C_1$-$C_6$) alkyl group, a substituted phenoxy ($C_1$-$C_6$) alkyl group with 1 to 5 substituents on the ring which may be the same or different and are selected from Y (Y is described below), a phenyl group, a substituted phenyl group with 1 to 5 substituents which may be the same or different and are selected from Y (Y is described below), a heterocyclic group (wherein the heterocyclic group is a pyridyl group, a pyridine-N-oxide group, a pyrimidinyl group, a pyradinyl group, a triadinyl group, a furyl group, a tetrahydrofuryl group, a thienyl group, a tetrahydrothienyl group, a tetrahydropyranyl group, a tetrahydrothiopyranyl group, an oxazolyl group, a isoxazolyl group, an oxadiazolyl group, a thiazolyl group, an isothiazolyl group, a thiadiazolyl group, an imidazolyl group, a triazolyl group, a pyrazolyl group or a pyrolydinyl group), a substituted heterocyclic group (wherein the heterocyclic group is the same as described above) with one or more substituents which may be the same or different and are selected from Y (Y is described below), a ($C_1$-$C_6$) alkoxycarbonyl group, a mono ($C_1$-$C_6$) alkylaminocarbonyl group, a di ($C_1$-$C_6$) alkylaminocarbonyl group in which the alkyl groups may be the same or different, a hydroxyl group or a cyano group; and $R^5$ and $R^6$, $R^5$ and $R^7$, $R^5$ and $R^8$, $R^6$ and $R^7$, $R^6$ and $R^8$, and $R^7$ and $R^8$, may be mutually bonded to form a 3- to 7-membered ring, $R^5$ and $R^6$, and $R^7$ and $R^8$ may form a carbonyl group, and $R^6$ and $R^7$ may form a double bond;

A is an oxygen atom or a sulfur atom;

W is an oxygen atom or a sulfur atom;

X may be the same or different and is 1 to 4 substituents selected from the group consisting of a hydrogen atom, a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkoxy ($C_1$-$C_6$) alkyl group, a halo ($C_1$-$C_6$) alkoxy ($C_1$-$C_6$) alkyl group, a ($C_1$-$C_6$) alkylthio group, a halo ($C_1$-$C_6$) alkylthio group, a ($C_1$-$C_6$) alkylthio ($C_1$-$C_6$) alkyl group, a halo ($C_1$-$C_6$) alkylthio ($C_1$-$C_6$) alkyl group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a phenyl group, a substituted phenyl group with 1 to 5 substituents which may be the same or different and are selected from Y (Y is described below), a phenoxy group, a substituted phenoxy group with 1 to 5 substituents which may be the same or different and are selected from Y (Y is described below), a phenylthio group, a substituted phenylthio group with 1 to 5 substituents which may be the same or different and are selected from Y (Y is described below), a phenylsulfinyl group, a substituted phenylsulfinyl group with 1 to 5 substituents which may be the same or different and are selected from Y (Y is described below), a phenylsulfonyl group, a substituted phenylsulfonyl group with 1 to 5 substituents which may be the same or different and are selected from Y (Y is described below), a ($C_1$-$C_6$) alkylcarbonyl group, a halo ($C_1$-$C_6$) alkylcarbonyl group, a phenylcarbonyl group, a substituted phenylcarbonyl group with 1 to 5 substituents which may be the same or different and are selected from Y (Y is described below), a ($C_1$-$C_6$) alkoxycarbonyl group,

a carboxyl group, mono ($C_1$-$C_6$) alkylaminocarbonyl group, a di ($C_1$-$C_6$) alkylaminocarbonyl group in which the alkyl groups may be the same or different, a phenylaminocarbonyl group, a substituted phenylaminocarbonyl group with 1 to 5 substituents which may be the same or different and are selected from Y (Y is described below), a phenyl ($C_1$-$C_6$) alkylaminocarbonyl group, a substituted phenyl ($C_1$-$C_6$) alkylaminocarbonyl group with 1 to 5 substituents which may be the same or different and are selected from Y (Y is described below), a hydroxyl group and a cyano group; X, together with an adjacent carbon atom on the benzene ring, may also form a 5- or 6-membered ring with a ($C_1$-$C_4$) alkylene group which may be interrupted by 1 or 2 hetero atoms which may be the same or different and are selected from the group consisting of an oxygen atom, a sulfur atom and a nitrogen atom (wherein the nitrogen atom may be substituted with a hydrogen atom, a ($C_1$-$C_6$) alkyl group, a ($C_2$-$C_6$) alkenyl group, a ($C_2$-$C_6$) alkynyl group or a cyclo ($C_3$-$C_6$) alkyl group);

Y may be the same or different and is 1 to 5 substituents selected from the group consisting of a halogen atom; a ($C_1$-$C_6$) alkyl group; a ($C_2$-$C_6$) alkenyl group; a ($C_2$-$C_6$) alkynyl group; a cyclo ($C_3$-$C_6$) alkyl group; a halo ($C_1$-$C_6$) alkyl group; a cyclohalo ($C_3$-$C_6$) alkyl group; a ($C_1$-$C_6$) alkoxy group; a halo ($C_1$-$C_6$) alkoxy group; a ($C_1$-$C_6$) alkylthio group; a halo ($C_1$-$C_6$) alkylthio group; a ($C_1$-$C_6$) alkylsulfinyl group; a halo ($C_1$-$C_6$) alkylsulfinyl group; a ($C_1$-$C_6$) alkylsulfonyl group; a halo ($C_1$-$C_6$) alkylsulfonyl group; a phenyl group; a substituted phenyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a heterocyclic group (wherein the heterocyclic group is the same as described above); a substituted heterocyclic group (wherein the heterocyclic group is the same as described above) with one or more substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenoxy group; a substituted phenoxy group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenylthio group; a substituted phenylthio group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenylsulfinyl group; a substituted phenylsulfinyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo ($C_1$-$C_6$) alkyl group, a cyclohalo ($C_3$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group, a halo ($C_1$-$C_6$) alkylsulfonyl group, a ($C_1$-$C_6$) alkylcarbonyl group, a halo ($C_1$-$C_6$) alkylcarbonyl group, a ($C_1$-$C_6$) alkoxycarbonyl group, a carboxyl group, a mono ($C_1$-$C_6$) alkylaminocarbonyl group, a di ($C_1$-$C_6$) alkylaminocarbonyl group in which the alkyl groups may be the same or different, a hydroxyl group and a cyano group; a phenylsulfonyl group; a substituted phenylsulfonyl group with 1 to 5 substituents which may be the same or different and are selected from the group consisting of a halogen atom, a ($C_1$-$C_6$) alkyl group, a ($C_2$-$C_6$) alkenyl group, a ($C_2$-$C_6$) alkynyl group, a cyclo ($C_3$-$C_6$) alkyl group, a halo

$(C_1-C_6)$ alkyl group, a cyclohalo $(C_3-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group, a $(C_1-C_6)$ alkylcarbonyl group, a halo $(C_1-C_6)$ alkylcarbonyl group, a $(C_1-C_6)$ alkoxycarbonyl group, a carboxyl group, a mono $(C_1-C_6)$ alkylaminocarbonyl group, a di $(C_1-C_6)$ alkylaminocarbonyl group in which the alkyl groups may be the same or different, a hydroxyl group and a cyano group; a $(C_1-C_6)$ alkylcarbonyl group; a halo $(C_1-C_6)$ alkylcarbonyl group; a phenylcarbonyl group; a substituted phenylcarbonyl group with 1 to 5 substituents which may be the same or different and are selected from the group consisting of a halogen atom, a $(C_1-C_6)$ alkyl group, a $(C_2-C_6)$ alkenyl group, a $(C_2-C_6)$ alkynyl group, a cyclo $(C_3-C_6)$ alkyl group, a halo $(C_1-C_6)$ alkyl group, a cyclohalo $(C_3-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group, a $(C_1-C_6)$ alkylcarbonyl group, a halo $(C_1-C_6)$ alkylcarbonyl group, a $(C_1-C_6)$ alkoxycarbonyl group, a carboxyl group, a mono $(C_1-C_6)$ alkylaminocarbonyl group, a di $(C_1-C_6)$ alkylaminocarbonyl group in which the alkyl groups may be the same or different, a hydroxyl group and a cyano group; a $(C_1-C_6)$ alkoxycarbonyl group; a carboxyl group; a mono $(C_1-C_6)$ alkylaminocarbonyl group; a di $(C_1-C_6)$ alkylaminocarbonyl group in which the alkyl groups may be the same or different; a phenylaminocarbonyl group; a substituted phenylaminocarbonyl group with 1 to 5 substituents which may be the same or different and are selected from the group consisting of a halogen atom, a $(C_1-C_6)$ alkyl group, a $(C_2-C_6)$ alkenyl group, a $(C_2-C_6)$ alkynyl group, a cyclo $(C_3-C_6)$ alkyl group, a halo $(C_1-C_6)$ alkyl group, a cyclohalo $(C_3-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group, a $(C_1-C_6)$ alkylcarbonyl group, a halo $(C_1-C_6)$ alkylcarbonyl group, a $(C_1-C_6)$ alkoxycarbonyl group, a carboxyl group, a mono $(C_1-C_6)$ alkylaminocarbonyl group, a di $(C_1-C_6)$ alkylaminocarbonyl group in which the alkyl groups may be the same or different, a hydroxyl group and a cyano group; a phenyl $(C_1-C_6)$ alkylaminocarbonyl group; a substituted phenyl $(C_1-C_6)$ alkylaminocarbonyl group with 1 to 5 substituents which may be the same or different and are selected from the group consisting of a halogen atom, a $(C_1-C_6)$ alkyl group, a $(C_2-C_6)$ alkenyl group, a $(C_2-C_6)$ alkynyl group, a cyclo $(C_3-C_6)$ alkyl group, a halo $(C_1-C_6)$ alkyl group, a cyclohalo $(C_3-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group, a $(C_1-C_6)$ alkylcarbonyl group, a halo $(C_1-C_6)$ alkylcarbonyl group, a $(C_1-C_6)$ alkoxycarbonyl group, a carboxyl group, a mono $(C_1-C_6)$ alkylaminocarbonyl group, a di $(C_1-C_6)$ alkylaminocarbonyl group in which the alkyl groups may be the same or different, a hydroxyl group and a cyano group; a hydroxyl group; and a cyano group, and, Y, together with an adjacent carbon atom or nitrogen atom on the benzene ring or heterocyclic ring, may form 5-or 6-membered ring with a $(C_1-C_4)$ alkylene group which may be interrupted by 1 or 2 hetero atoms which may be the same or different and are selected from the group consisting of an oxygen atom, a sulfur atom and a nitrogen atom (wherein the nitrogen atom may be substituted with a hydrogen atom, a $(C_1-C_6)$ alkyl group, a $(C_2-C_6)$ alkenyl group, a $(C_2-C_6)$ alkynyl group or a cyclo $(C_3-C_6)$ alkyl group), or a salt thereof.

**2.** The haloalkyl sulfonanilide derivative or salt thereof according to claim 1, wherein $R^1$, $R^2$, $R^5$, $R^6$, $R^7$, $R^8$, A, W and Y are the same as in claim 1; $R^3$ and $R^4$ are hydrogen atoms; and X may be the same or different and is 1 to 4 substituents selected from the group consisting of a hydrogen atom, a halogen atom, a $(C_1-C_6)$ alkyl group, a cyclo $(C_3-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkoxy $(C_1-C_6)$ alkyl group, a halo $(C_1-C_6)$ alkoxy $(C_1-C_6)$ alkyl group, a $(C_1-C_6)$ alkylthio group, a halo $(C_1-C_6)$ alkylthio group, a $(C_1-C_6)$ alkylthio $(C_1-C_6)$ alkyl group, a halo $(C_1-C_6)$ alkylthio $(C_1-C_6)$ alkyl group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group.

**3.** The haloalkyl sulfonanilide derivative or a salt thereof according to claim 1,
wherein $R^2$, $R^5$, $R^6$, $R^7$, $R^8$, A and W are the same as in claim 1,

$R^1$ is a fluoro $(C_1-C_6)$ alkyl group;
$R^3$ and $R^4$ are hydrogen atoms;
X may be the same or different and is 1 to 4 substituents selected from the group consisting of a hydrogen atom, a halogen atom, a $(C_1-C_6)$ alkyl group, a halo $(C_1-C_6)$ alkyl group, an alkoxy $(C_1-C_6)$ group, a haloalkoxy $(C_1-C_6)$, and a $(C_1-C_6)$ alkoxy $(C_1-C_6)$ alkyl group;
Y may be the same or different and is 1 to 5 substituents selected from the group consisting of a halogen atom; a $(C_1-C_6)$ alkyl group; a cyclo $(C_3-C_6)$ alkyl group; a halo $(C_1-C_6)$ alkyl group; a $(C_1-C_6)$ alkoxy group; a halo

$(C_1-C_6)$ alkoxy group; a $(C_1-C_6)$ alkylthio group; a halo $(C_1-C_6)$ alkylthio group, a $(C_1-C_6)$ alkylsulfinyl group; a halo $(C_1-C_6)$ alkylsulfinyl group; a $(C_1-C_6)$ alkylsulfonyl group; a halo $(C_1-C_6)$ alkylsulfonyl group; a phenyl group; a substituted phenyl group with 1 to 5 substituents which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a heterocyclic group (wherein the heterocyclic group is the same as in claim 1); a substituted heterocyclic group (wherein the heterocyclic group is the same as in claim 1) with 1 to 5 substituents which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a phenoxy group; a substituted phenoxy group with 1 to 5 substituents which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a phenylthio group; a substituted phenylthio group with 1 to 5 substituents which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a $(C_1-C_6)$ alkylcarbonyl group; a halo $(C_1-C_6)$ alkylcarbonyl group; a phenylcarbonyl group; a substituted phenylcarbonyl group with 1 to 5 substituents which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a $(C_1-C_6)$ alkoxycarbonyl group; a carboxyl group; a mono $(C_1-C_6)$ alkylaminocarbonyl group; a di $(C_1-C_6)$ alkylaminocarbonyl group in which the alkyl groups may be the same or different; a phenylaminocarbonyl group; a substituted phenylaminocarbonyl group with 1 to 5 substituents on the ring which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; a phenyl $(C_1-C_6)$ alkylaminocarbonyl group; a substituted phenyl $(C_1-C_6)$ alkylaminocarbonyl group with 1 to 5 substituents on the ring which may be the same or different and are selected from the group consisting of a halogen atom, a $(C_1-C_6)$ alkyl group, a halo $(C_1-C_6)$ alkyl group, a $(C_1-C_6)$ alkoxy group, a halo $(C_1-C_6)$ alkoxy group, a $(C_1-C_6)$ alkylthio group, a halo $(C_1-C_6)$ alkylthio group, a $(C_1-C_6)$ alkylsulfinyl group, a halo $(C_1-C_6)$ alkylsulfinyl group, a $(C_1-C_6)$ alkylsulfonyl group, a halo $(C_1-C_6)$ alkylsulfonyl group and a cyano group; and a cyano group; and Y, together with an adjacent carbon atom or nitrogen atom on the benzene ring or heterocyclic ring, may form 5- or 6-membered ring with a $(C_1-C_4)$ alkylene group which may be interrupted by 1 or 2 hetero atoms, which may be the same or different and are selected from the group consisting of a oxygen atom, a sulfur atom and a nitrogen atom (wherein the nitrogen atom may be substituted with a hydrogen atom, a $(C_1-C_6)$ alkyl group, a $(C_2-C_6)$ alkenyl group, a $(C_2-C_6)$ alkynyl group or a cyclo $(C_3-C_6)$ alkyl group).

**4.** A herbicide comprising as an active ingredient the haloalkyl sulfonanilide derivative or salt thereof according to any one of claims 1 to 3.

**5.** A method for using a herbicide, comprising treating a soil or plant with an effective dose of the herbicide according to claim 4.

**INTERNATIONAL SEARCH REPORT**

International application No.
PCT/JP2006/303307

A. CLASSIFICATION OF SUBJECT MATTER
***C07D263/16***(2006.01), ***C07D263/22***(2006.01), ***C07D263/24***(2006.01), ***C07D263/52***(2006.01), ***C07D263/58***(2006.01), ***C07D277/14***(2006.01), ***A01N43/76***(2006.01), ***A01N43/78***(2006.01), ***A01N47/02***(2006.01)

According to International Patent Classification (IPC) or to both national classification and IPC

B. FIELDS SEARCHED

Minimum documentation searched (classification system followed by classification symbols)
A01N43/76, A01N43/78, A01N47/02, C07D263/16, C07D263/22, C07D263/24, C07D263/52, C07D263/58, C07D277/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2004-107323 A (Nihon Nohyaku Co., Ltd.), 08 April, 2004 (08.04.04), Claims; compound No.12-1 & WO 2004/11429 A1 | 1-5 |
| A | US 4367090 A (Rhone-Poulenc Agrochimie), 04 January, 1983 (04.01.83), Full text (Family: none) | 1-5 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 March, 2006 (16.03.06) | 11 April, 2006 (11.04.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004107322 A
- JP 58183603 A


### Non-patent literature cited in the description


- *Tetrahedron Lett,* 1990, vol. 31, 4661
- *Synth. Commun.,* 1994, vol. 24 (10), 1415
- *Bull. Soc. Chim. Fr.,* 1943, vol. 10, 347
- *J. Am. Chem. Soc,* 1945, vol. 67, 522-523